# EUROPEAN PATENT APPLICATION

(11) **EP 4 585 141 A1**
(43) Date of publication of application: **16.07.2025**
(21) Application number: 23863013.1
(22) Date of filing: 29.08.2023
(51) Int. Cl.: A61B 3/103

(54) **OPHTHALMIC INFORMATION PROCESSING DEVICE, OPHTHALMIC DEVICE, OPHTHALMIC INFORMATION PROCESSING METHOD, AND PROGRAM**

(30) Priority: 09.09.2022 JP 2022143373
(71) Applicant: TOPCON CORPORATION, Tokyo 174-8580 (JP)
(72) Inventor: TATARA, Yoko, Tokyo 174-8580 (JP)
(74) Representative: Gagel, Roland
(86) International application number: PCT/JP2023/031116
(87) International publication number: WO 2024/053474

(57) **Abstract**

This ophthalmic information processing device calculates the refractive power of an examined eye wearing an intraocular lens on the basis of wavefront aberration information obtained by conducting wavefront aberration measurement for the examined eye. The ophthalmic information processing device comprises an acquisition unit and a calculation unit. The acquisition unit acquires at least intraocular lens information representing the optical characteristics of the intraocular lens. The calculation unit uses the intraocular lens information to change a refractive power calculation process method and calculate the refractive power of the examined eye.

## Description

### [TECHNICAL FIELD]

The present invention relates to an ophthalmic information processing apparatus, an ophthalmic apparatus, an ophthalmic information processing method, and a program.

### [BACKGROUND ART]

When a cataract progresses, it is common for cataract surgery to be performed. In the cataract surgery, a content of a lens capsule is removed and an intraocular lens (IOL, hereinafter) is implanted in the lens capsule. There are various types of IOLs. The examinee needs to select an appropriate type of IOL, taking into the contrast of image, the brightness, the distant visual acuity, the near visual acuity, etc. After the surgery, the dioptric power of the eye to be examined wearing the IOL is measured, and the view and/or the recovery of visual acuity vision and other factors are checked.

There have been several proposals for ophthalmic apparatuses for examining the eye to be examined wearing such an IOL. For example, Patent Document 1 discloses a method of acquiring a transillumination image of the eye to be examined and of determining from the acquired transillumination image whether or not the IOL is worn by the eye to be examined. For example, Patent Document 2 discloses a method of obtaining a dioptric power using a point image group of a part of point images obtained using wavefront aberration information. For example, Patent Document 3 discloses a method of measuring a dioptric power of an eye to be examined wearing the IOL by projecting a ring pattern.

### [PRIOR ART DOCUMENTS]

### [PATENT DOCUMENTS]

[Patent Document 1] Japanese Unexamined Patent Publication No. 2014-209994
[Patent Document 2] Japanese Unexamined Patent Publication No. 2017-213124
[Patent Document 3] Japanese Unexamined Patent Publication No. 2021-083940

### [SUMMARY OF THE INVENTION]

### [PROBLEMS TO BE SOLVED BY THE INVENTION]

In the conventional methods, the dioptric power is calculated using a uniform method regardless of the type of IOL. Therefore, there is a problem that the reliability of the calculation result of the dioptric power decreases depending on the type of IOL implanted in the eye to be examined.

The present invention has been made in view of such circumstances, and one of objects of the present invention is to provide a new technique for improving reliability of calculation result of a dioptric power of an eye to be examined wearing an IOL.

### [MEANS OF SOLVING THE PROBLEMS]

One aspect of embodiments is an ophthalmic information processing configured to calculate a dioptric power of an eye to be examined based on wavefront aberration information obtained by performing wavefront aberration measurement on the eye to be examined wearing an intraocular lens. The ophthalmic information processing apparatus includes an acquisition unit configured to acquire intraocular lens information representing at least optical characteristics of the intraocular lens; and a calculator configured to change calculation processing method of the dioptric power in accordance with the intraocular lens information to calculate the dioptric power of the eye to be examined.

Another aspect of the embodiments is an ophthalmic apparatus including a measurement optical system including a focusing lens and configured to measure wavefront aberration of the eye to be examined; and the ophthalmic information processing apparatus described above.

Still another aspect of the embodiments is an ophthalmic information processing method for calculating a dioptric power of an eye to be examined based on wavefront aberration information obtained by performing wavefront aberration measurement on the eye to be examined wearing an intraocular lens. The ophthalmic information processing method includes an acquisition step of acquiring intraocular lens information representing at least optical characteristics of the intraocular lens; and a calculation step of changing calculation processing method of the dioptric power in accordance with the intraocular lens information to calculate the dioptric power of the eye to be examined.

Still another aspect of the embodiments is a program of causing a computer to execute each step of the ophthalmic information processing method described above.

### [EFFECTS OF THE INVENTION]

According to the present invention, a new technique for improving reliability of calculation result of a dioptric power of an eye to be examined wearing an IOL can be provided.

### [BRIEF EXPLANATION OF THE DRAWINGS]

[FIG. 1] FIG. 1 is a schematic diagram illustrating an example of a configuration of an optical system of an ophthalmic apparatus according to a first embodiment.
[FIG. 2] FIG. 2 is a schematic diagram illustrating an example of the configuration of the optical system of the ophthalmic apparatus according to the first embodiment.
[FIG. 3] FIG. 3 is a schematic diagram illustrating an example of the configuration of the optical system of the ophthalmic apparatus according to the first embodiment.
[FIG. 4] FIG. 4 is a schematic diagram illustrating an example of the configuration of the optical system of the ophthalmic apparatus according to the first embodiment.
[FIG. 5] FIG. 5 is an explanatory diagram of an operation of the ophthalmic apparatus according to the first embodiment.
[FIG. 6] FIG. 6 is a schematic diagram illustrating an example of a configuration of a processing system of the ophthalmic apparatus according to the first embodiment.
[FIG. 7] FIG. 7 is a schematic diagram illustrating an example of a configuration of the processing system of the ophthalmic apparatus according to the first embodiment.
[FIG. 8] FIG. 8 is a schematic diagram illustrating an example of a configuration of the processing system of the ophthalmic apparatus according to the first embodiment.
[FIG. 9] FIG. 9 is a schematic diagram illustrating an example of a configuration of the processing system of the ophthalmic apparatus according to the first embodiment.
[FIG. 10] FIG. 10 is a schematic diagram for explaining a multifocal refractive type IOL according to the first embodiment.
[FIG. 11] FIG. 11 is a schematic diagram for explaining a multifocal refractive type IOL according to the first embodiment.
[FIG. 12] FIG. 12 is a schematic diagram illustrating an example of a configuration of the processing system of the ophthalmic apparatus according to the first embodiment.
[FIG. 13] FIG. 13 is a schematic diagram for explaining a multifocal diffractive type IOL according to the first embodiment.
[FIG. 14] FIG. 14 is a schematic diagram illustrating an example of a configuration of the processing system of the ophthalmic apparatus according to the first embodiment.
[FIG. 15] FIG. 15 is a schematic diagram for explaining an extended depth of focus type IOL according to the first embodiment.
[FIG. 16] FIG. 16 is a flowchart illustrating an example of an operation of the ophthalmic apparatus according to the first embodiment.
[FIG. 17] FIG. 17 is a flowchart illustrating an example of an operation of the ophthalmic apparatus according to the first embodiment.
[FIG. 18] FIG. 18 is a flowchart illustrating an example of an operation of the ophthalmic apparatus according to the first embodiment.
[FIG. 19] FIG. 19 is a flowchart illustrating an example of an operation of the ophthalmic apparatus according to the first embodiment.
[FIG. 20] FIG. 20 is a flowchart illustrating an example of an operation of the ophthalmic apparatus according to the first embodiment.
[FIG. 21] FIG. 21 is a flowchart illustrating an example of an operation of the ophthalmic apparatus according to the first embodiment.
[FIG. 22] FIG. 22 is an explanatory diagram of an operation of the ophthalmic apparatus according to the first embodiment.
[FIG. 23] FIG. 23 is an explanatory diagram of an operation of the ophthalmic apparatus according to the first embodiment.
[FIG. 24] FIG. 24 is an explanatory diagram of an operation of the ophthalmic apparatus according to the first embodiment.
[FIG. 25] FIG. 25 is an explanatory diagram of an operation of the ophthalmic apparatus according to the first embodiment.
[FIG. 26] FIG. 26 is a flowchart illustrating an example of an operation of the ophthalmic apparatus according to a second embodiment.
[FIG. 27] FIG. 27 is a flowchart illustrating an example of an operation of the ophthalmic apparatus according to the second embodiment.

### [MODES FOR CARRYING OUT THE INVENTION]

Referring now to the drawings, exemplary embodiments of an ophthalmic information processing apparatus, an ophthalmic apparatus, an ophthalmic information processing method, and a program according to the present invention are described below. Any of the contents of the documents cited in the present specification and arbitrary known techniques may be applied to the embodiments below.

An ophthalmic information processing apparatus is configured to calculate a dioptric power of an eye to be examined based on wavefront aberration information obtained by performing wavefront aberration measurement on the eye to be examined wearing an IOL (intraocular lens). For example, by implanting the IOL in a lens capsule whose contents have been surgically removed, the eye to be examined can wear the IOL. The ophthalmic information processing apparatus is configured to acquire IOL (intraocular lens) information representing at least optical characteristics of the IOL, and to change calculation processing method of the dioptric power in accordance with the intraocular lens information to calculate the dioptric power of the eye to be examined.

The IOL information includes, for example, at least one of information representing the number of focal points (the number of focal point distances) of the IOL, information representing positions of areas with different focal point distances in the IOL, information representing whether or not to utilize the refractive phenomenon of light, information representing whether or not to utilize the diffraction phenomenon of light, or information representing whether or not to have a deep depth of focus. Such IOL information can be identified by a predetermined type of IOL. Examples of the type of IOL include a monofocal type and a multifocal type. Examples of the multifocal type include a multifocal diffractive type and a multifocal refractive type. In some embodiments, the multifocal type includes an Extended of Depth of Focus (hereinafter referred to as EDoF) type. Such IOL information is obtained by designated by a user (examinee, examiner, doctor, etc.) using an operation unit or by analyzing an anterior segment image or a transillumination image of the eye to be examined wearing the IOL to determine the type of IOL.

The ophthalmic information processing apparatus is configured to calculate the dioptric power according to the type of IOL using a calculation processing method according to the IOL information, such as changing the application method of the known Zernike polynomial approximation using wavefront aberration information including a Hartmann image according to the IOL information. The wavefront aberration information includes the Hartmann image acquired in a focused state with respect to the eye to be examined, corresponding to an average focal point distance of the intraocular lens, or a plurality of Hartmann images acquired in a focused state with respect to the eye to be examined, corresponding to each of a plurality of focal point distances of the intraocular lens. The dioptric power includes, for example, a spherical power (S), a cylindrical power (C), and an astigmatic axis angle (A). In some embodiments, the ophthalmic information processing apparatus is configured to calculate the dioptric power for each focal point distance of the IOL.

Further, according to the type of IOL, at least one of a plurality of point images that make up the acquired Hartmann image is separated into two or more separated point images. Each of the two or more separated point images corresponds to the focal point distance of the IOL. Therefore, the ophthalmic information processing apparatus is configured to classify the two or more separated point images, each of which corresponds to each point image, into any one of the two or more point image groups based on the IOL information, and to calculate the dioptric power using a known method in that a Zernike polynomial approximation is performed for each point image group.

In some embodiments, the ophthalmic information processing apparatus is configured to acquire pupil diameter information representing a pupil diameter of the eye to be examined, and to calculate the dioptric power based on the wavefront aberration information within a region demarcated based on the acquired pupil diameter information. For example, the ophthalmic information processing apparatus is configured to normalize the wavefront aberration information using the pupil diameter information, and to calculate the dioptric power using a known method in that a Zernike polynomial approximation is performed using the normalized wavefront aberration information.

Thereby, the dioptric power can be obtained by changing a calculation processing method (calculation method) in accordance with the type worn by the eye to be examined. As a result, the reliability of the calculation result of the dioptric power of the eye to be examined wearing an IOL can be improved.

The ophthalmic apparatus according to the embodiments realizes the function(s) of the ophthalmic information processing apparatus according to the embodiments. An ophthalmic information processing method according to the embodiments includes one or more steps for realizing the processing executed by a processor (computer) in the ophthalmic information processing apparatus according to the embodiments. A program according to the embodiments causes the processor to execute each step of the ophthalmic information processing method according to the embodiments. A recording medium (storage medium) according to the embodiments is a computer readable non-transitory recording medium (storage medium) on which the program according to the embodiments is recorded.

The term "processor" as used herein refers to a circuit such as, for example, a central processing unit (CPU), a graphics processing unit (GPU), an application specific integrated circuit (ASIC), and a programmable logic device (PLD). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program.

Hereinafter, the ophthalmic apparatus according to the embodiments will be described as having the functions of the ophthalmic information processing apparatus according to the embodiments. Further, the types of the IOL are assumed to include the monofocal type, the multifocal type, and the EDoF type. In this case, unless otherwise mentioned, a case where the number of focal points of the multifocal type IOL is "2" will be described. However, the configuration according to the embodiments can be applied to a case where the number of focal points is three or more.

The ophthalmic apparatus according to the embodiments can perform at least one of arbitrary subjective inspections or arbitrary objective measurements. In the subjective inspections, information (optotypes, etc.) is presented to the examinee, and the result is obtained based on a response to the information from the examinee. Examples of the subjective inspection include a visual field test, and a subjective refractivity measurement such as a distant test, a reading test, a contrast test, a glare test. In the objective measurements, light is projected onto an eye to be examined and information on the eye to be examined is acquired based on detection result(s) of returning light thereof. The objective measurements include a measurement for acquiring the characteristics of the eye to be examined and a photographing for acquiring an image of the eye to be examined. Examples of the objective measurement include an objective refractometry, a corneal shape measurement, an intraocular pressure measurement, a fundus photographing, a tomographic imaging using optical coherence tomography (hereinafter, OCT) (OCT imaging), and a measurement using OCT.

Hereafter, it is assumed that the ophthalmic apparatus according to the embodiments is an apparatus that can perform subjective inspection such as distant test, and reading test, and can also perform objective measurement such as objective refractometry using wavefront aberration measurement and corneal shape measurement. However, the configuration of the ophthalmic apparatus according to the embodiments is not limited to this.

### <First embodiment>

### [Configuration]

The ophthalmic apparatus according to the first embodiment includes a face support unit fixed to a base, and a stage movable in front, back, upward, downward, left, and right directions relative to the base. The stage is provided with a head unit in which an optical system for performing inspection (measurement) of the eye to be examined is housed. The face support unit and the head unit can be relatively moved by operating with respect to an operation unit provided on the side of an examiner. Further, in the ophthalmic apparatus, the face support unit and the head unit can be relatively moved automatically by performing alignment described below.

### (Optical system)

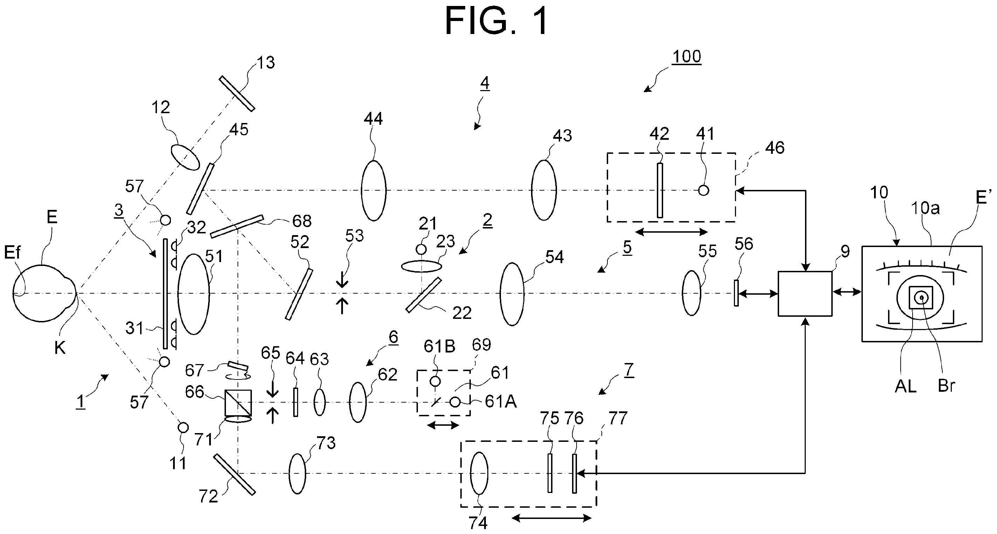

FIG. 1 illustrates an example of a configuration of an optical system of the ophthalmic apparatus according to the first embodiment. The ophthalmic apparatus 100 according to the first embodiment includes, as an optical system for performing inspections of an eye E to be examined, a Z alignment system 1, an XY alignment system 2, a keratometry system 3, an optotype projection system 4, an observation system 5, an aberration measurement projection system 6, and an aberration measurement light reception system 7. In addition, the ophthalmic apparatus includes a processor 9.

### (Processor 9)

The processor 9 controls each part of the ophthalmic apparatus. Further, the processor 9 is capable of performing various types of arithmetic processing. The processor 9 includes a processor. The processor 9 realizes the functions according to the embodiments, for example, by reading out computer program(s) stored in a storage circuit or a storage device and executing the computer program(s).

### (Observation System 5)

The observation system 5 is configured to photograph a moving image of an anterior segment of the eye E to be examined. For example, returning light from the anterior segment of the eye E to be examined, which is illuminated by light (for example, infrared light with a central wavelength of 950 nm) from a plurality of anterior segment illumination light sources 57 arranged at positions away from an optical axis of the observation system 5, passes through an objective lens 51, is transmitted through a dichroic mirror 52, and passes through an opening of a diaphragm 53. The light that has passed through the opening of the diaphragm 53 is transmitted through a half mirror 22, passes through a relay lens 54, and is guided to an imaging lens 55. The imaging lens 55 forms an image of the light, that has been guided from the relay lens 54, on a light receiving surface of an area sensor (image sensor) 56. The light receiving surface of the area sensor 56 is disposed at a position substantially conjugate optically to a pupil of the eye E to be examined. The area sensor 56 performs photographing and a signal outputting at a predetermined rate. The output (video signal) of the area sensor 56 is input to the processor 9. The processor 9 displays an anterior segment image E' based on this video signal on a display screen 10a of a display unit 10. The anterior segment image E' is an infrared moving image, for example.

### (Z alignment system 1)

The Z alignment system 1 is configured to project light (infrared light) for performing alignment in an optical axis direction (front-back directions, Z direction) of the observation system 5 onto the eye E to be examined. Light emitted from a Z alignment light source 11 is irradiated onto a cornea K of the eye E to be examined, is reflected on the cornea K, and is guided to an imaging lens 12. The imaging lens 12 forms an image of the light, that has been guided, on a light receiving surface of a line sensor 13. When a position of a corneal apex changes in the front-back directions, a projected position of the light onto the line sensor 13 changes. The output of the line sensor 13 is input to the processor 9. The processor 9 obtains the position of the corneal apex of the eye E to be examined based on the projected position of the light on the line sensor 13, and performs Z alignment based on this position.

### (XY alignment system 2)

The XY alignment system 2 is configured to irradiate light (infrared light) for performing alignment in directions (left-right directions (X direction), up-down directions (Y direction)) orthogonal to the optical axis direction of the observation system 5 onto the eye E to be examined. The XY alignment system 2 includes an XY alignment light source 21 disposed on an optical path that is branched from the observation system 5 by the half mirror 22. Light emitted from the XY alignment light source 21 passes through the relay lens 23, and is reflected by the half mirror 22. The light that has been reflected by the half mirror 22 is focused on a front focal position of the objective lens 51 on the optical axis of the observation system 5. The focused light is transmitted through the dichroic mirror 52, is made into collimated light by the objective lens 51, and is irradiated onto the cornea K of the eye E to be examined. The light reflected on a surface of the cornea K forms a Purkinje image near a reflection focal point on the corneal surface of the eye E to be examined. The XY alignment light source 21 is disposed at a position substantially conjugate optically to the focal position of the objective lens 51. Reflected light from the cornea K is guided to the area sensor 56 through the observation system 5. On the light receiving surface of the area sensor 56, an image Br, that is caused by the Purkinje image (bright spot) of the light emitted from the XY alignment light source 21, is formed.

The processor 9 displays an alignment mark AL and the anterior segment image E' including the bright spot image Br on the display screen 10a, as shown in FIG. 1. In case of performing XY alignment manually, an examiner performs a movement operation of the optical system so as to guide the bright spot image Br into the alignment mark AL. In case of performing XY alignment automatically, the processor 9 controls a mechanism for moving the optical system so as to cancel a displacement of the bright spot image Br with respect to the alignment mark AL.

### (Keratometry system 3)

The keratometry system 3 is configured to project a ring-shaped light flux (infrared light) for measuring a curvature of the cornea K onto the cornea K. A keratometry plate 31 is disposed in the vicinity of the objective lens 51. A keratometry ring light source 32 is provided on the back side (the objective lens 51 side) of the keratometry plate 31. By illuminating the keratometry plate 31 with light from the keratometry ring light source 32, the ring-shaped light flux is projected onto the cornea K. Reflected light (keratometry ring image) of the ring-shaped light flux) is detected by the area sensor 56 along with the anterior segment image. The processor 9 calculates a corneal shape parameter, by performing a known calculation based on this keratometry ring image. A placido ring plate consisting of a plurality of rings may be disposed instead of the keratometry ring. In this case, not only the curvature of the cornea, but also a corneal shape can be measured.

### (Optotype projection system 4)

The optotype projection system 4 is configured to present various kinds of optotypes such as a fixation target and an optotype for a subjective inspection to the eye E to be examined. An optotype chart 42 displays a pattern representing an optotype, under the control from the processor 9. Light (visible light) emitted from a light source 41 passes through the optotype chart 42, passes through a relay lens 43 and a field lens 44, is reflected by a reflective mirror 45, is transmitted through a beam splitter 68, and is reflected by the dichroic mirror 52. Light reflected by the dichroic mirror 52 passes through the objective lens 51 and is projected onto the fundus Ef.

A movement unit 46 including the light source 41 and the optotype chart 42 is movable along an optical axis of the optotype projection system 4. A position of the movement unit 46 is adjusted so that the optotype chart 42 and the fundus Ef are substantially conjugate optically to each other.

The optotype chart 42 can display the pattern representing the fixation target for fixating the eye E to be examined under the control from the processor 9. A fixation position can be moved by sequentially changing a display position of the pattern representing the fixation target in the optotype chart 42, a visual line of the eye to be examined can be guided, and/or an accommodation of the eye to be examined can be induced. Examples of such optotype chart 42 include an electronic display device using a liquid crystal panel or an electroluminescence (EL), and a device (turret type) that places any one of a plurality of optotypes drawn on a rotating glass plate, etc. on the optical axis as appropriate. Further, the optotype projection system 4 may include a glare test optical system for projection glare light onto the eye E to be examined along with the optotype described above.

In case of performing the subjective inspection, the processor 9 controls the movement unit 46 based on the result of the objective measurement. The processor 9 causes the optotype selected by the examiner or the processor 9 to be displayed on the optotype chart 42. Thereby, the optotype is presented to the examinee. The examinee responses with respect to the optotype. Upon receiving input of the response contents, the processor 9 performs further control or calculates a subjective inspection value. For example, in the visual acuity measurement, the processor 9 selects a next optotype based on the response to the Landolt ring or the like, presents the next optotype to the eye to be examined, and determines the visual acuity value by repeatedly performing this.

In the objective measurement (objective refractometry, etc.), a landscape chart is projected on the fundus Ef. Alignment is performed while causing the examinee to fixate the landscape chart, and the dioptric power is measured in a state where fogging is promoted.

### (Aberration measurement projection system 6, Aberration measurement light reception system 7)

The aberration measurement projection system 6 and the aberration measurement light reception system 7 are used for the measurement of the ocular aberration characteristics of the eye E to be examined. The aberration measurement projection system 6 is configured to project light flux (mainly, infrared light) for the measurement of the ocular aberration characteristics onto the fundus Ef. The aberration measurement light reception system 7 is configured to receive returning light of the light flux from the fundus Ef of the eye E to be examined. The ocular aberration characteristics of the eye E to be examined are obtained from light receiving result of the returning light acquired by the aberration measurement light reception system 7.

The aberration measurement projection system 6 includes a light source 61 that can output light in two or more wavelength regions with different central wavelengths. The light source 61 may be configured with a single light source that can change the wavelength region (central wavelength) of the output light. Alternatively, the light source 61 may be configured to switch between two or more light sources that output light with different wavelength regions (central wavelengths) from each other. In FIG. 1, it is assumed that the light source 61 includes a light source 61A that outputs light in a first wavelength region including a first central wavelength and a light source 61B that outputs light in a second wavelength region including a second central wavelength. For example, the first central wavelength is 560 nm (visible region) and the second central wavelength is 840 nm (near infrared region). In this case, the light source 61 outputs light from any one of the light source 61A and the light source 61B. In some embodiments, an optical path from the light source 61A and an optical path from the light source 61B are coupled by a dichroic mirror, and the light source 61A and the light source 61B are exclusively controlled to be turned on. In some embodiments, the optical path from the light source 61A and the optical path from the light source 61B are coupled by the dichroic mirror, a first shutter that can be inserted into or be removed is provided between the light source 61A and the dichroic mirror, and a second shutter that can be inserted into or be removed is provided between the light source 61B and the dichroic mirror.

For each of the light sources (point light sources) 61A and 61B, a light source that emits a minute point-like light is used. Examples of the light sources 61A and 61B include, for example, a SLD (Super Luminescent Diode) with high converging performance. However, an LD (laser diodes) with high converging performance or an LED with small emission diameter and high luminance may also be used.

The movement unit 69 including the light source 61 is movable along an optical axis of the aberration measurement projection system 6. The light source 61 is disposed at a position substantially conjugate optically to the fundus Ef. Light (measurement light) emitted from the light source 61 passes through a relay lens 62 and a field lens 63, and is transmitted through a polarizer 64. The polarizer 64 transmits the s-polarized component alone among the polarized components of the light emitted from the light source 61. The light transmitted through the polarizer 64 passes through an opening of a diaphragm 65, is reflected by a polarization beam splitter 66 that reflects the s-polarized component, passes through a rotary prism 67, and is reflected by a beam splitter 68. Light reflected by the beam splitter 68 is reflected by the dichroic mirror 52, passes through the objective lens 51, and is projected onto the fundus Ef.

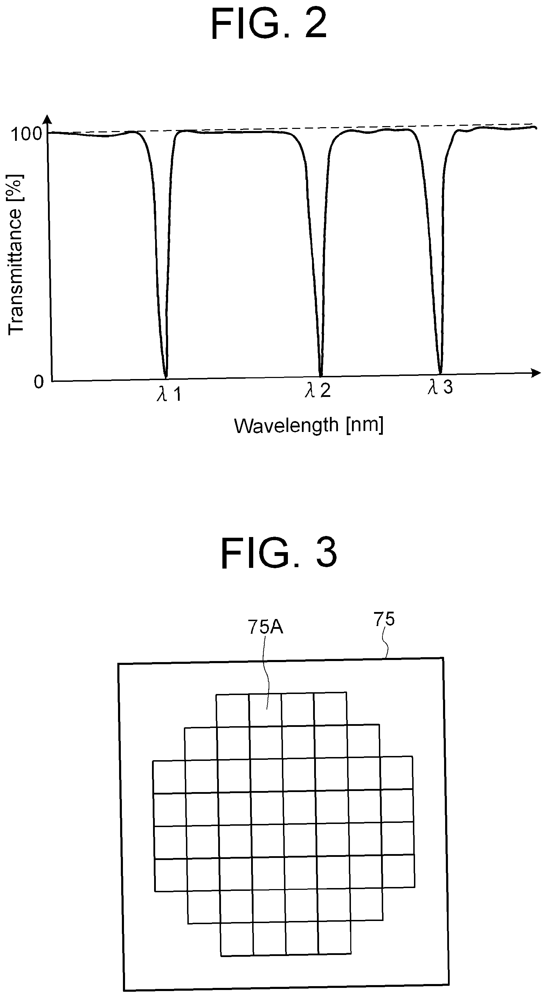

FIG. 2 schematically shows an example of wavelength selection characteristics of the beam splitter 68. **In** FIG. 2, a horizontal axis represents a transmittance of light, while the vertical axis represents wavelength.

For example, the beam splitter 68 reflects light in the wavelength region with the first wavelength λ1 as the central wavelength, light in the wavelength region with the second wavelength λ2 as the central wavelength, and light in the wavelength region with the third wavelength λ3 (0<λ1<λ2<λ3) as the central wavelength, and transmits light in other wavelength regions. For example, the first wavelength λ1 is the central wavelength (560nm) of light emitted from the light source 61A, the second wavelength λ2 is the central wavelength (840nm) of light emitted from the light source 61B, and the third wavelength λ3 is the central wavelength (950nm) of light emitted from the anterior segment illumination light source 57.

Thereby, the beam splitter 68 can transmit the light from the optotype projection system 4, and can reflect the light from the light sources 61A and 61B in the aberration measurement projection system 6 and the returning light of the light from the light sources 61A and 61B. As a result, the wavelength separation between the optotype projection system 4, and the aberration measurement projection system 6 and aberration measurement light reception system 7 can be performed well. Such beam splitter 68 may be a mirror with wavelength selectivity disclosed in, for example, Japanese Unexamined Patent Application Publication No. 2010-099354.

It should be noted that, without placing a light source at the position of the light source 61, the light from the light source 61 may be guided to the relay lens 62 through an optical fiber that connects the light source and the ophthalmic apparatus. **In** this case, a fiber end of the optical fiber is disposed at a position substantially conjugate optically to the fundus Ef.

The rotary prism 67 is used for averaging the unevenness of reflectance on the blood vessels and/or the disease site of the fundus Ef, or for reducing the speckle noise caused by the SLD light source.

Light incident on the eye E to be examined no longer maintains its polarization state due to scattered reflection caused by the fundus. Therefore, the returning light from the fundus Ef becomes mixed light with the p-polarized component and the s-polarized component. Such returning light from the fundus Ef passes through the objective lens 51, and is reflected by the dichroic mirror 52 and the beam splitter 68. The returning light reflected by the beam splitter 68 passes through the rotary prism 67, and is guided to the polarization beam splitter 66. The polarization beam splitter 66 transmits the s-polarized component alone among the polarized components of the returning light. The p-polarized component of the light transmitted through the polarization beam splitter 66 passes through the field lens 71, is reflected by a reflective mirror 72, passes through a relay lens 73, and is guided to a movement unit 77. Light that is regularly reflected on a surface of the objective lens 51 or the cornea K of the eye E to be examined keeps the s-polarized. Therefore, the regularly reflected light is reflected by the polarization beam splitter 66, and does not enter the aberration measurement light reception system 7. Thereby, the occurrence of ghost can be reduced.

The movement unit 77 includes a collimator lens 74, a Hartmann plate 75, and an area sensor 76. The collimator lens 74 functions as a focusing lens through the movement of the movement unit 77. As the area sensor 76, for example, a CCD (Charge Coupled Device) image sensor or a CMOS (Complementary Metal Oxide Semiconductor) image sensor is used. Light that has been guided to the movement unit 77 passes through the collimator lens 74, and enters the Hartmann plate 75. The Hartmann plate 75 is disposed at a position substantially conjugate optically to the pupil of the eye E to be examined. The movement unit 77 is movable along an optical axis of the aberration measurement light reception system 7. The movement unit 77 is moved along the optical axis according to the dioptric power of the eye E to be examined so that the fundus Ef and the front focal point of the collimator lens 74 are substantially conjugate optically to each other.

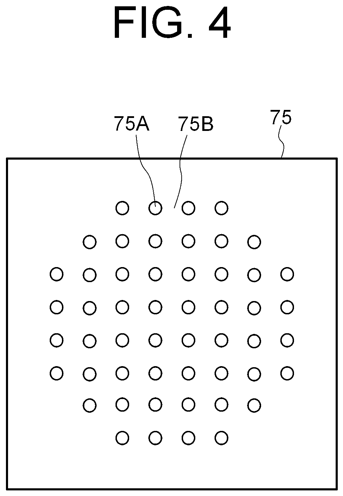

FIG. 3 and FIG. 4 show explanatory diagrams of the Hartmann plate 75 according to the embodiments. FIG. 3 and FIG. 4 schematically represent a configuration of the Hartmann plate 75 when viewed from the optical axis direction of the aberration measurement light reception system 7.

The Hartmann plate 75 generates a plurality of converging light from the returning light from the fundus Ef. As shown in FIG. 3 and FIG. 4, in the Hartmann plate 75, a plurality of microlenses 75A is arranged in a reticular pattern. The Hartmann plate 75 splits the incident light into many light fluxes, and then collects each of the light fluxes.

For example, the Hartmann plate 75 has a configuration in which the microlenses 75A are arranged on a glass plate by etching or molding, as shown in FIG. 3. **In** this case, the opening of each of the microlens can be larger, and the signal intensity can be increased.

Alternatively, as shown in FIG. 4, the Hartmann plate 75 may have a configuration in which the microlenses 75A are arranged with a light shielding portion 75B by forming a chromium light shielding film or the like around each microlens 75A. The microlenses 75A are not limited to those squarely arranged. The microlenses 75A may also be those concentrically arranged, those arranged at each apex position of a triangle, or those arranged in a hexagonal close-packed pattern.

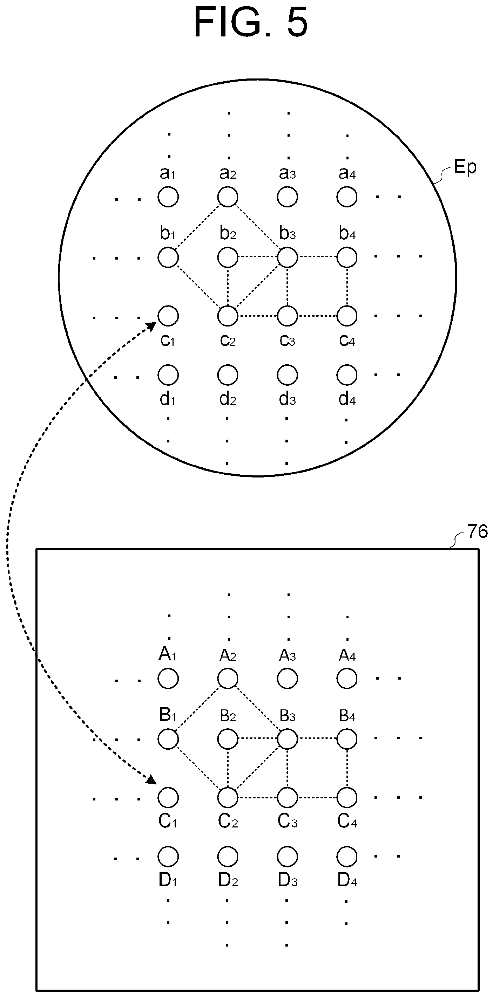

The area sensor 76 is disposed at the focal positions of the microlenses 75A, and detects light (converging light) that is respectively converged by the Hartmann plate 75. As shown in FIG. 5, on the light receiving surface of the area sensor 76, point images A₁, ..., B₁, ..., C₁, ... are formed by the microlenses 75A of the Hartmann plate 75 corresponding to irradiated regions a₁, ..., b₁, ..., c₁, ... of light on the pupil Ep of the eye E to be examined. **In** the case where the fundus Ef and the front focal point of the collimator lens 74 are in a substantially optically conjugate relationship as described above, intervals between the positions of the center of gravity of the point images formed on the light receiving surface of the area sensor 76 (or between peak positions of the brightness of the point images) are approximately equal to the distance between the lens centers of the microlenses 75A. The area sensor 76 detects a group of point images formed by microlenses 75A in the Hartmann plate 75. The processor 9 acquires detection signals based on the point image groups detected by area sensor 76 and the positional information indicating the detection positions of the point image groups, and analyzes the position of the point image formed by each microlens 75A to obtain the wavefront aberration of the light that has been entered into the Hartmann plate 75. Thereby, the ocular aberration characteristics of the eye E to be examined are obtained from the distances between the point images. The processor 9 obtains the dioptric power of the eye E to be examined from the obtained ocular aberration characteristics.

The processor 9 can move the movement unit 69 and the movement unit 77 in the optical axis direction, respectively, based on the calculated dioptric power, so that the light source 61 (light sources 61A and 61B), the fundus Ef, and the front focal position of the collimator lens 74 are conjugate optically to each other. Further, the processor 9 can move the movement unit 46 in the optical axis direction thereof, in conjunction with the movement of the movement units 69 and 77.

**In** some embodiments, the ophthalmic apparatus 100 can acquire transillumination images of the eye E to be examined. For example, the transillumination image can be acquired by turning one (or a part) of a plurality of anterior segment illumination light sources 57 on, projecting light onto the fundus Ef from a position away from the optical axis through the pupil, and receiving returning light of the light with the area sensor 56.

In some embodiments, the transillumination image is acquired by turning the XY alignment light source 21 on, projecting the light onto the fundus Ef through the pupil, and receiving the returning light of the light with the area sensor 56. In this case, the XY alignment light source 21 may be an SLD or a high-brightness LED. For example, the XY alignment light source 21 may be configured to output light from an LD as an alignment light source in performing alignment and to output light from the SLD or the high-brightness LED as an illumination light source for acquiring a transillumination image in acquiring transillumination images.

### (Configuration of processing system)

A processing system of the ophthalmic apparatus 100 according to the embodiments will be described.

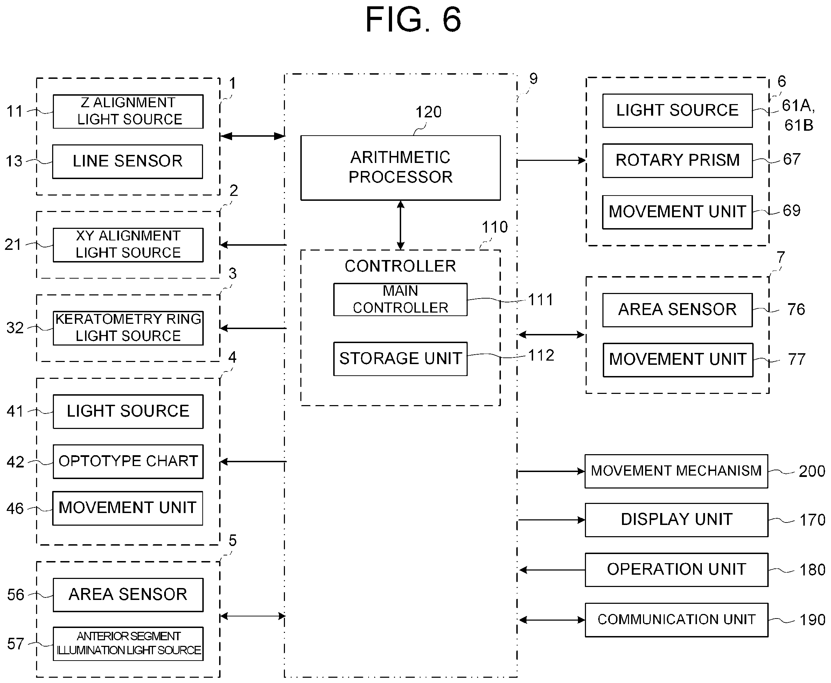

FIG. 6 shows an example of a functional configuration of a processing system of the ophthalmic apparatus 100. FIG. 6 illustrates an example of a functional block diagram of the processing system of the ophthalmic apparatus according to the embodiments. In FIG. 6, like parts are designated by like reference numerals as in FIG. 1 and repetitious description of such parts may not be provided.

The processor 9 includes a controller 110 and an arithmetic processor 120. Further, the ophthalmic apparatus 100 includes a display unit 170, an operation unit 180, a communication unit 190, and a movement mechanism 200.

The movement mechanism 200 is a mechanism for moving the head unit in front, back, upward, downward, left and right directions. Here, the head unit houses the optical systems such as the Z alignment system 1, the XY alignment system 2, the keratometry system 3, the optotype projection system 4, the observation system 5, the aberration measurement projection system 6, and the aberration measurement light reception system 7. For example, the movement mechanism 200 is provided with an actuator that generates a driving force for moving the movement mechanism 200 and a transmission mechanism that transmits the driving force from the actuator to the movement mechanism 200. The actuator includes a pulse motor, for example. The transmission mechanism includes a combination of gears, a rack and pinion, and the like, for example. The controller 110 (main controller 111) performs control for the movement mechanism 200 by sending a control signal to the actuator.

### (Controller 110)

The controller 110 includes a processor and controls each part of the ophthalmic apparatus. The controller 110 includes a main controller 111 and a storage unit 112. The storage unit 112 stores, in advance, a computer program for controlling the ophthalmic apparatus. The computer program includes program(s) for controlling light source, program(s) for controlling sensor, program(s) for controlling optical system, program(s) for arithmetic processing, program(s) for user interface, and the like. The main controller 111 operates according to the computer programs, and thereby the controller 110 performs the control process.

The main controller 111 performs various controls of the ophthalmic apparatus, as a measurement controller. Examples of control for the Z alignment system 1 include control of the Z alignment light source 11, control of the line sensor 13. Examples of the control of the Z alignment light source 11 include turning on and off of the light source, and adjustment of light quantity. Examples of the control for the line sensor 13 include adjustment of exposure of a detecting element, adjustment of gain of the detecting element, and adjustment of detecting rate of the detecting element. Thereby, the Z alignment light source 11 is switched between lighting and non-lighting or the light quantity is changed. The main controller 111 captures a signal detected by the line sensor 13 and identifies a projected position of light onto the line sensor 13 based on the captured signal. The main controller 111 obtains a position of a corneal apex of the eye E to be examined based on the identified projected position and controls the movement mechanism 200 based on the identified position to move the head unit in front and back directions (Z alignment).

Examples of control for the XY alignment system 2 include control of the XY alignment light source 21. Examples of the control of the Z alignment light source 21 include turning on and off of the light source, and adjustment of light quantity. Thereby, the XY alignment light source 21 is switched between lighting and non-lighting, or the light quantity is changed. The main controller 111 captures a signal detected by the area sensor 56, and identifies a position of a bright spot image on the basis of returning light of the light from the XY alignment light source 21 based on the captured signal. The main controller 111 controls the movement mechanism 200 to move the head unit in left, right, upward, downward directions so as to cancel a displacement the position of the bright spot image with respect to a predetermined target position (for example, a center position of the alignment mark) (XY alignment).

Examples of control for the keratometry system 3 include control of the keratometry ring light source 32. Examples of the control of the keratometry ring light source 32 include turning on and off of the light source, and adjustment of light quantity. Thereby, the keratometry ring light source 32 is switched between lighting and non-lighting, or the light quantity is changed. The main controller 111 controls the arithmetic processor 120 to perform a known calculation on a keratometry ring image detected by the area sensor 56. Thereby, corneal shape parameter(s) of the eye E to be examined is/are obtained.

Examples of the control for the optotype projection system 4 include control of the light source 41, control of the optotype chart 42, and movement control of the movement unit 46. Examples of the control for the light source 41 include turning the light source on and off, and adjustment of light quantity. Thereby, the light source 41 is switched between lighting and non-lighting, or the light quantity is changed. Examples of the control of the optotype chart 42 include displaying on and off of the optotypes and/or the fixation target, and switching the display position of the fixation target. Thereby, the optotypes and/or the fixation target are/is projected onto the fundus Ef of the eye E to be examined. For example, the optotype projection system 4 include a movement mechanism that moves the movement unit 46 in the optical axis direction. As is the case with the movement mechanism 200, this movement mechanism is provided with an actuator that generates driving force for moving the movement mechanism and a transmission mechanism that transmits the driving force from the actuator to the movement unit 46. The main controller 111 controls the movement mechanism by sending a control signal to the actuator to move the movement unit 46 in the optical axis direction. Thereby, a position of the movement unit 46 is adjusted so that the optotype chart 42 and the fundus Ef are conjugate optically to each other.

Examples of the control for the observation system 5 include control of the area sensor 56, and control of the anterior segment illumination light source 57. Examples of the control of the area sensor 56 include adjustment of exposure of the area sensor 56, adjustment of gain of the area sensor 76, and adjustment of detecting rate of the area sensor 76. The main controller 111 captures signal(s) detected by the area sensor 56, and controls the arithmetic processor 120 to perform processing such as forming images based on the captured signal(s). Examples of control of the anterior segment illumination light source 57 include turning on and off the light source, adjustment of light quantity. Thereby, the anterior segment illumination light source 57 is switched between lighting and non-lighting, one or a part of the anterior segment illumination light sources 57 is turned on, or the light quantity of each light source is changed.

Examples of control for the aberration measurement projection system 6 include control of the light sources 61A and 61B, control of the rotary prism 67, control of the movement unit 69. Examples of the control for the light sources 61A and 61B include turning the light sources on and off, and adjustment of light quantity. Thereby, the light sources 61A and 61B are switched between lighting and non-lighting, the light quantity is changed, the wavelength region of the light emitted from the light source 61 is changed. Examples of the control of the rotary prism 67 include control of rotating the rotary prism 67. For example, a rotary mechanism that rotates the rotary prism 67 is provided and the main controller 111 controls this rotary mechanism to rotate the rotary prism 67. For example, the aberration measurement projection system 6 include a movement mechanism that moves the movement unit 69 in the optical axis direction. As is the case with the movement mechanism 200, this movement mechanism is provided with an actuator that generates driving force for moving the movement mechanism and a transmission mechanism that transmits the driving force from the actuator to the movement unit 69. The main controller 111 controls the movement mechanism by sending a control signal to the actuator to move the movement unit 69 in the optical axis direction.

Examples of the control for the aberration measurement light reception system 7 include control of the area sensor 76, and movement control of the movement unit 77. Examples of the control of the area sensor 76 include adjustment of exposure of the area sensor 76, adjustment of gain of the area sensor 76, and adjustment of detecting rate of the area sensor 76. The main controller 111 captures signal(s) detected by the area sensor 76, and controls the arithmetic processor 120 to perform calculation processing of the ocular aberration characteristics based on the captured signal(s). For example, the aberration measurement light reception system 7 include a movement mechanism that moves the movement unit 77 in the optical axis direction. As is the case with the movement mechanism 200, this movement mechanism is provided with an actuator that generates driving force for moving the movement mechanism and a transmission mechanism that transmits the driving force from the actuator to the movement unit 77. The main controller 111 controls the movement mechanism by sending a control signal to the actuator to move the movement unit 77 in the optical axis direction. The main controller 111 controls the movement mechanism by sending a control signal to the actuator to move the movement unit 77 in the optical axis direction.

The main controller 111 can, as a display controller, display various kinds of information on the display unit 170. Examples of the information displayed on the display unit 170 include a result of the objective measurement (aberration measurement result) and a result of the subjective inspection acquired by using the above optical system, and an image or information based on these. For example, the dioptric power obtained by the arithmetic processor 120, a wavefront aberration map representing the distribution of wavefront aberration(s), a simulation image representing the view, the visual acuity simulation result(s), or the like are displayed on the display unit 170. The main controller 111 can display these information for each area of the focal point distances of the IOL, or can identifiably display a part of the information.

Further, the main controller 111 performs a process of writing data in the storage unit 112 and a process of reading out data from the storage unit 112.

### (Storage unit 112)

The storage unit 112 stores various types of data. Examples of the data stored in the storage unit 112 include inspection result(s) of the subjective inspection, measurement result(s) of the objective measurement, image data of the anterior segment image, image data of the Hartmann point image, information on eye to be examined, and processing result(s) obtained by the arithmetic processor 120. The information on the eye to be examined includes information on the examinee such as patient ID and name, and information on the eye to be examined such as identification information of the left/right eye. Further, the storage unit 112 stores various types of programs and data to run the ophthalmic apparatus.

### (Arithmetic processor 120)

The arithmetic processor 120 includes a processor and executes the processing of each of the following parts according to the program(s) stored in a storage unit (not shown) (or storage unit 112).

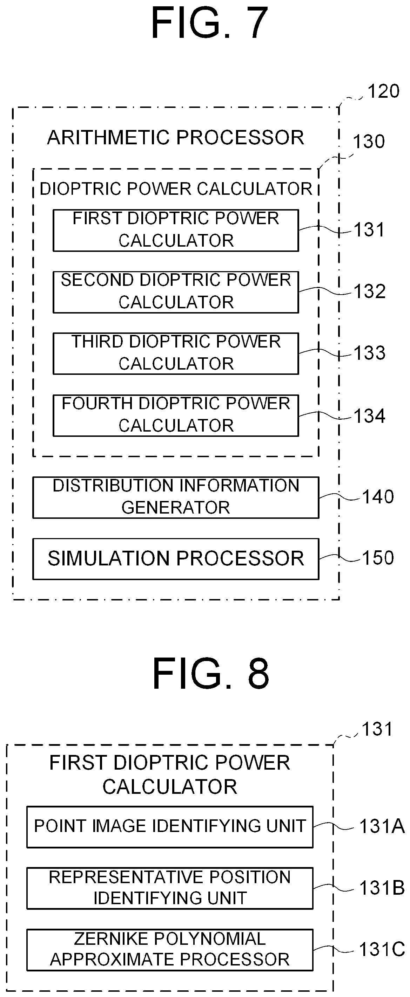

FIG. 7 shows a functional block diagram of an example of a configuration of the arithmetic processor 120 in FIG. 6.

The arithmetic processor 120 includes a dioptric power calculator 130, a distribution information generator 140, and a simulation processor 150.

### (Dioptric power calculator 130)

The dioptric power calculator 130 obtains the dioptric power of the eye E to be examined using a calculation processing method in accordance with the type of IOL. The types of IOL include a monofocal type, a multifocal refractive type, a multifocal diffractive type, and an EDoF type. Therefore, the dioptric power calculator 130 includes a first dioptric power calculator 131, a second dioptric power calculator 132, a third dioptric power calculator 133, and a fourth dioptric power calculator 134.

The first dioptric power calculator 131 performs first dioptric power calculation processing that calculates the dioptric power of the eye E to be examined using a calculation processing method corresponding to the monofocal type IOL. The second dioptric power calculator 132 performs second dioptric power calculation processing that calculates the dioptric power of the eye E to be examined using a calculation processing method corresponding to the multifocal refractive type IOL. The third dioptric power calculator 133 performs third dioptric power calculation processing that calculates the dioptric power of the eye E to be examined using a calculation processing method corresponding to the multifocal diffractive type IOL. The fourth dioptric power calculator 134 performs fourth dioptric power calculation processing that calculates the dioptric power of the eye E to be examined using a calculation processing method corresponding to the EDoF type IOL.

The functional block that makes up each of the first dioptric power calculator 131, the second dioptric power calculator 132, the third dioptric power calculator 133, and the fourth dioptric power calculator 134 may be shared as appropriate, in case that it has the same function.

### (First dioptric power calculator 131)

When the IOL worn by the eye E to be examined is the monofocal type IOL, the first dioptric power calculator 131 calculates a single dioptric power based on the wavefront aberration information including the Hartmann image acquired by the wavefront aberration measurement.

FIG. 8 shows a functional block diagram of an example of a configuration of the first dioptric power calculator 131 shown in FIG. 7.

The first dioptric power calculator 131 includes a point image identifying unit 131A, a representative position identifying unit 131B, and a Zernike polynomial approximate processor 131C.

The point image identifying unit 131A identifies the point images that make up the Hartmann image. The point image identifying unit 131A identifies the point images based on the brightness values of the Hartmann image acquired by the area sensor 56. In some embodiments, enhancement processing of the point image(s) is performed on the Hartmann image, before the point images are identified by the point image identifying unit 131A.

The representative position identifying unit 131B identifies a representative position of the point image identified by the point image identifying unit 131A. Examples of the representative position include a position of the center of gravity, a center position, a position in the point image closest to the center of the Hartmann image, and a position in the point image farthest from the center of the Hartmann image. In the present embodiment, the representative position identifying unit 131B identifies the position of the center of gravity, as the representative position.

The Zernike polynomial approximate processor 131C performs Zernike polynomial approximate processing based on the representative positions of the point images identified by the representative position identifying unit 131B, and obtains the spherical power S, the astigmatic power C and the astigmatic axis angle A as the single dioptric power. In other words, the Zernike polynomial approximate processor 131C obtains slopes of light beams at the representative positions of the point images identified by the representative position identifying unit 131B, and obtains an approximate expression for the wavefront by a known calculation using the obtained amounts of the slopes of the light beams. The obtained approximate expression for the wavefront is expressed by Zernike coefficients and a Zernike polynomial. The wavefront aberration information is represented by the Zernike coefficients. In this case, the Zernike polynomial approximate processor 131C can normalize the wavefront aberration information using the pupil diameter of the eye E to be examined or the pupil diameter of the schematic eye, as disclosed in Japanese Unexamined Patent Application Publication No. 2002-209854, for example. The Zernike polynomial approximate processor 131C obtains the spherical power S, the astigmatic power C and the astigmatic axis angle A from the low-order term(s) of the Zernike coefficients, using a known calculation. For example, the Zernike polynomial approximate processor 131C can calculate the dioptric power using a method disclosed in Japanese Unexamined Patent Application Publication No. 2002-209854 or Japanese Unexamined Patent Application Publication No. 2017-213124.

### (Second dioptric power calculator 132)

When the IOL worn by the eye E to be examined is the multifocal refractive type IOL, the second dioptric power calculator 132 calculates a plurality of dioptric powers, each of which corresponds to each of a plurality of focal point distances of the IOL, based on the wavefront aberration information including the Hartmann image acquired by the wavefront aberration measurement. In other words, the second dioptric power calculator 132 calculates a plurality of dioptric powers including the dioptric power corresponding to a far point and the dioptric power corresponding to a near point, for each area corresponding to the focal point distance of the IOL.

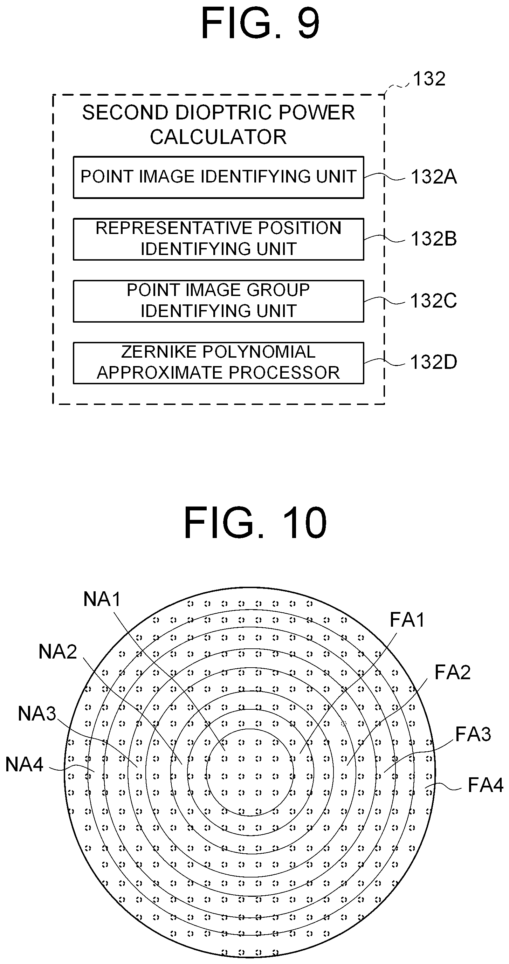

FIG. 9 shows a functional block diagram of an example of a configuration of the second dioptric power calculator 132 shown in FIG. 7.

The second dioptric power calculator 132 includes a point image identifying unit 132A, a representative position identifying unit 132B, and a point image group identifying unit 132C, and a Zernike polynomial approximate processor 132D.

The point image identifying unit 132A identifies the point images that make up the Hartmann image, in the same way as the point image identifying unit 131A. In some embodiments, enhancement processing of the point image(s) is performed on the Hartmann image, before the point images are identified by the point image identifying unit 132A.

The representative position identifying unit 132B identifies a representative position (position of the center of gravity) of the point image identified by the point image identifying unit 132A, in the same way as the representative position identifying unit 131B.

The point image group identifying unit 132C classifies two or more separated point images, each of which is generated by separating the point image that should has originally formed, into point image groups for each focal point distance of the IOL (according to focal point of distance of the IOL).

The Zernike polynomial approximate processor 132D calculates the dioptric power of the eye E to be examined in the same way as the Zernike polynomial approximate processor 131C, for each point image group identified by the point image group identifying unit 132C.

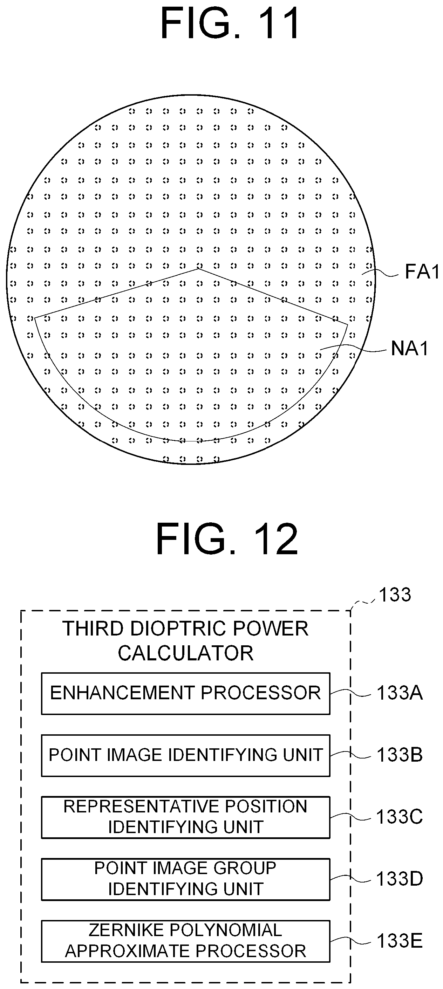

FIG. 10 and FIG. 11 schematically show point images acquired by the area sensor 56, each of the point images corresponding to the lens areas of the multifocal refractive type IOL according to the first embodiment. FIG. 10 represents the point images corresponding to a toric (annular) type multifocal refractive type IOL. In the toric type, areas with different focal point distances are arranged concentrically. FIG. 11 represents the point images corresponding to a sector type multifocal refractive type IOL. In the sector type, areas with different focal point distances are arranged below the lens area.

In the case that the number of the focal points of the toric type multifocal refractive type IOL is "2", for example, near (near vision, reading) areas, which can focus on near objects, and far (far vision, distant) areas, which can focus on the distance, are arranged alternately, from the center to the outside. In FIG. 10, from the center to the outside, a near area NA1, a far area FA1, a near area NA2, a far area FA2, ..., a near area NA4, and a far area FA4 are arranged alternately. In the case that the number of the focal points of the toric type multifocal refractive type IOL is "3", similarly, the near areas, medium (medium vision) areas, which can focus on intermediate distance between the near object and the distance, and the far areas are arranged in order, from the center to the outside. In the case that the number of the focal points of the toric type multifocal refractive type IOL is 4 or more, similarly, the near areas, two or more medium areas with intermediate distances different from each other, and the far areas are arranged in order, from the center to the outside. In the present example, the center side is arranged as the near area and the area far from the center is arranged as the far area. However, the center side may be arranged as the far area and the area far from the center may be arranged as the near area.

The second dioptric power calculator 132 identifies the point image group included in the area corresponding to a predetermined focal point distance, and calculates the dioptric powers for each point image group, based on the wavefront aberration information obtained from the identified point image groups.

In the case that the number of the focal points of the sector type multifocal refractive type IOL is "2", as shown in FIG. 11, a near area NA1 is arranged below the lens area where the entire area is a far area FA1. In the case that the number of the focal points of the sector type multifocal refractive type IOL is 3 or more, for example, each of one or more medium areas are arranged between the far area FA1 and the near area NA1.

In this case, the second dioptric power calculator 132 identifies the point image group included in the area corresponding to a focal point distance, and calculates the dioptric powers for each point image group, based on the wavefront aberration information obtained from the identified point image groups, in the same way as the toric type.

Thereby, for example, the dioptric power can be calculated from the point image group(s) included in the near area, and the dioptric power can be calculated from the point image group(s) included in the far area. In some embodiments, the dioptric power can be calculated from the point image groups, each of which is included in each of the one or more medium areas.

### (Third dioptric power calculator 133)

When the IOL worn by the eye E to be examined is the multifocal diffractive type IOL, the third dioptric power calculator 133 calculates a plurality of dioptric powers, each of which corresponds to each of a plurality of focal point distances of the IOL, based on the wavefront aberration information including the Hartmann image acquired by the wavefront aberration measurement. Specifically, the third dioptric power calculator 133 classifies the two or more separated point images, each of which is generated by separating the point image that constitutes the Hartmann image, into the point image groups for each focal point distance of the IOL, and calculates a plurality of dioptric powers including a dioptric power corresponding to the far point and a dioptric power corresponding to the near point for each of focal point distances based on the classified two or more separated point images.

FIG. 12 shows a functional block diagram of an example of a configuration of the third dioptric power calculator 133 shown in FIG. 7.

The third dioptric power calculator 133 includes an enhancement processor 133A, a point image identifying unit 133B, a representative position identifying unit 133C, a point image group identifying unit 133D, and a Zernike polynomial approximate processor 133E.

The enhancement processor 133A performs enhancement processing of point image in the Hartmann image. For example, the enhancement processor 133A increases the contrast of the Hartmann image and removes portions where the brightness values are saturated.

The point image identifying unit 133B identifies the point images that constitute the Hartmann image, in the same way as the point image identifying unit 131A. Specifically, the point image identifying unit 133B identifies the two or more separated point images, each of which is generated by separating the point image that constitutes the Hartmann image. **In** this case, the point image identifying unit 133B identifies one or more separated point images on the far point side by analyzing the Hartmann image, and identifies the remaining one or more separated point images on the near point side by analyzing the Hartmann image on which the enhancement processing has been performed by the enhancement processor 133A. **In** other words, the point image identifying unit 133B identifies point images with a predetermined first brightness value or greater from the Hartmann image as the separated point images on the far point side, and identifies point images with a predetermined second brightness value or greater in the Hartmann image from which the portions with the saturated brightness values are removed by increasing contrast as the separated point images on the near point side. Thereby, the separated point images on the near point side, where the contrast is reduced caused by the multifocal diffractive type IOL, can be easily identified.

In some embodiments, the point image identifying unit 133B identifies one or more separated point images on the far point side and the one or more separated point images on the near point side by analyzing the Hartmann image on which the enhancement processing has been performed by the enhancement processor 133A.

In some embodiments, the wavefront aberration measurement is performed using light in the visible region (for example, light from light source 61A) in order to facilitate identification of the separated point images on the near point side.

The representative position identifying unit 133C identifies a representative position (position of the center of gravity) of the point image identified by the point image identifying unit 133B, in the same way as the representative position identifying unit 131B.

The point image group identifying unit 133D classifies the two or more separated point images, which are identified by the representative position identifying unit 133C, into any one of two or more point image groups corresponding to the focal point distances of the IOL. The point image group identifying unit 133D classifies each of the two or more separated point images, from which one point image is separated, into any one of a point image group of the separated point images of the near point, a point image group of one or more separated point images corresponding to one or more medium point, and a point image group of the separated point images of the far point, in order from closest to the center of the Hartmann image.

The Zernike polynomial approximate processor 133E calculates the dioptric power of the eye E to be examined in the same way as the Zernike polynomial approximate processor 131C, for each point image group identified by the point image group identifying unit 133D.

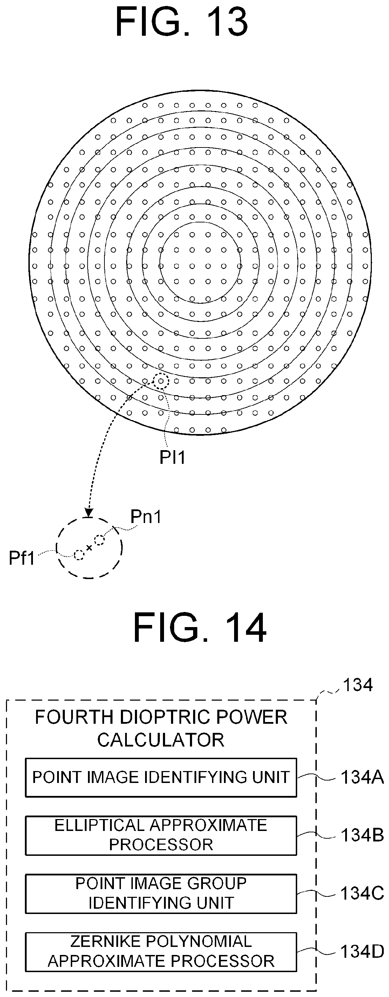

FIG. 13 schematically shows point images acquired by the area sensor 56, each of the point images corresponding to the lens areas of the multifocal diffractive type IOL according to the first embodiment. In FIG. 13, the number of the focal points is assumed to be "2".

The point image that constitutes the Hartmann image is separated into the two or more separated point images according to the focal point distance of the IOL. As shown in FIG. 13, when the number of focal points is "2", the point image PI1 is separated into a separated point image (point image at near point) Pn1 closer to the center of the Hartmann image and a separated point image (point image at far point) Pf1 farther from the center of the Hartmann image, with reference to the point image that should originally be formed. Depending on the configuration of the optical system, the point image PI1 may be separated such that the separated point image close to the center of the Hartmann image is point image Pf1 at far point, and a separated point image far from the center of the Hartmann image is a point image Pn1 at near point.

The third dioptric power calculator 133 classifies the identified two or more separated point images into any one of the two or more point image groups corresponding to the focal point distances of the IOL, and calculates the dioptric power for each point image group. In FIG. 13, the point image group identifying unit 133D classifies the two separated point image, each of which corresponds to each point image that makes up the Hartmann image, into the point image group of the near point and the point image group of the far point. The Zernike polynomial approximate processor 133E calculates the dioptric power for each point image group, based on the wavefront aberration information obtained from the classified point image groups. The third dioptric power calculator 133 can calculate the dioptric power for each point image group as described above, for each Hartmann image.

### (Fourth dioptric power calculator 134)

When the IOL worn by the eye E to be examined is the EDoF type IOL, the fourth dioptric power calculator 134 identifies the two separated point images corresponding to the point images that constitute the Hartmann image based on the wavefront aberration information including the Hartmann image acquired by the wavefront aberration measurement, classifies the identified separated point images into any one of the two point image groups, and calculates the dioptric power for each point image group based on the wavefront aberration information obtained from the classified point image groups. Specifically, the fourth dioptric power calculator 134 classifies two focal points of an approximate ellipse, which is identified by performing elliptical approximation on each of a plurality of point images that constitute the Hartmann image, into groups for each focal point distance, and calculates a plurality of dioptric powers including a dioptric power corresponding to the far point and a dioptric power corresponding to the near point for each of focal point distances based on the classified two or more separated point images.

FIG. 14 shows a functional block diagram of an example of a configuration of the fourth dioptric power calculator 134 shown in FIG. 7.

The fourth dioptric power calculator 134 includes a point image identifying unit 134A, an elliptical approximate processor 134B, a point image group identifying unit 134C, and a Zernike polynomial approximate processor 134D.

The point image identifying unit 134A identifies the point images that make up the Hartmann image, in the same way as the point image identifying unit 131A. In some embodiments, enhancement processing of the point image(s) is performed on the Hartmann image, before the point images are identified by the point image identifying unit 134A.

The elliptical approximate processor 134B identifies a plurality of approximate ellipses by performing know elliptical approximate processing on each of the plurality of point images identified by the point image identifying unit 134A, and identifies the two focal points of each of the plurality of identified approximate ellipses.

The point image group identifying unit 134C identifies the focal point closer to the center of the Hartmann image among the two focal points of the approximate ellipse identified by the elliptical approximate processor 134B as the point image at near point and the focal point farther from the center of the Hartmann image as the point image at far point. The point image group identifying unit 134C classifies the plurality of point images at near point and the plurality of point images at far point, which are identified for each of the plurality of approximate ellipses, into a point image group of the point image at near point and a point image group of the point image at far point.

The Zernike polynomial approximate processor 134D calculates the dioptric power of the eye E to be examined in the same way as the Zernike polynomial approximate processor 131C, for each point image group identified by the point image group identifying unit 134C.

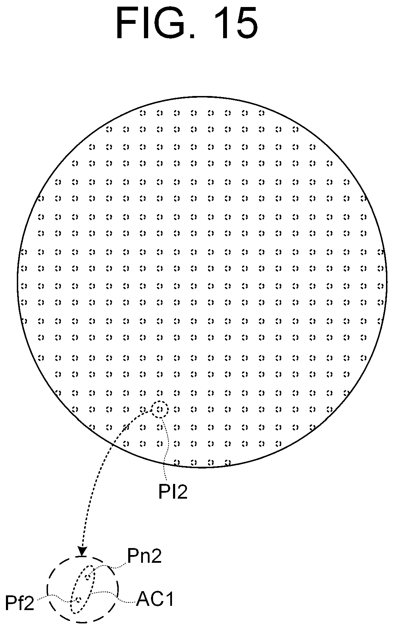

FIG. 15 schematically shows point images acquired by the area sensor 56, each of the point images corresponding to the lens areas of the EDoF type IOL according to the first embodiment.

The point image identifying unit 134A identifies the point images by analyzing the Hartmann image. In this case, the point image that constitutes the Hartmann image is an image that extends in a direction connecting the far point and the near point (blurred image) due to the deep depth of focus.

In the case where the point images are arranged in a toric shape or a sector shape similar to the multifocal refractive type IOL, the arithmetic processor 120 can calculate the dioptric power for each point image group similar to the multifocal refractive type IOL in the second dioptric power calculator 132. In the case where the point image is separated into the two or more separated point images similar to the multifocal diffractive type IOL, the arithmetic processor 120 can calculate the dioptric power for each point image group similar to the multifocal diffractive type IOL in the third dioptric power calculator 133.

In the case where a shape of the point image is an elliptical shape, the elliptical approximate processor 134B performs elliptical approximate processing on a shape of the point image PI2 to identify an approximate ellipse AC1, and identifies two focal points Pn2 and Pf2 of the identified approximate ellipse AC1. The point image group identifying unit 134C identifies the focal point Pn2 close to the center of the Hartmann image among the identified two focal points as the point image at near point and the focal point Pf2 far from the center of the Hartmann image as the point image at far point. Further, the point image group identifying unit 134C similarly classifies two focal points as the point image at near point and the point images at far point for each of the plurality of point images, and classifies the identified point images at near point and the identified point images at far point into a point image group of the point image at near point and a point image group of the point image at far point. The Zernike polynomial approximate processor 134D calculates the dioptric power for each point image group, based on the wavefront aberration information obtained from the classified point image groups.

Further, the dioptric power calculator 130 calculates a corneal dioptric power, a corneal astigmatism power, and a corneal astigmatic axis angle based on the keratometry ring image acquired by the observation system 5. For example, the dioptric power calculator 130 calculates a corneal curvature radius of the steepest meridian and/or the flattest meridian of the anterior surface of the cornea by analyzing the keratometry ring image and calculates above parameters based on the corneal curvature radius.

### (Distribution information generator 140)

The distribution information generator 140 generates distribution information (wavefront aberration map) representing distribution of the wavefront aberrations, for each focal point distance of the IOL. The distribution information generator 140 generates the distribution information based on the wavefront aberration information, as disclosed in Japanese Unexamined Patent Application Publication No. 2002-209854. When the horizontal direction in the pupil of the eye E to be examined (or the light receiving surface of the area sensor 56) is assumed to be the x direction and the vertical direction is assumed to be the y direction, the distribution information generator 140 interpolates the wavefront aberration at each position (x, y) in the horizontal direction and vertical direction using a known method to generate the distribution information representing the two-dimensional distribution of the wavefront aberrations.

When the IOL worn by the eye E to be examined is the monofocal type IOL, the distribution information generator 140 generates the distribution information representing the distribution of the wavefront aberrations based on single wavefront aberration information.

When the IOL worn by the eye E to be examined is the multifocal refractive type IOL or the multifocal diffractive type IOL, the distribution information generator 140 generates the distribution information representing the distribution of wavefront aberrations at the near point based on the wavefront aberration information obtained from the point image group on the nearest point side, and generates the distribution information representing the distribution of wavefront aberrations at the far point based on the wavefront aberration information obtained from the point image group on the farthest point side.

When the IOL worn by the eye E to be examined is the EDoF type IOL, the distribution information generator 140 generates the distribution information representing the distribution of wavefront aberrations at the near point based on the wavefront aberration information obtained from the point image group of the point images at near point, and generates the distribution information representing the distribution of wavefront aberrations at far point based on the wavefront aberration information obtained from the point image group of the point images at far point.

### (Simulation processor 150)

The simulation processor 150 performs a visual acuity simulation. For example, the simulation processor 150 obtains a plurality of images on the retinal surface, by performing ray tracing processing from object points converted from each of the plurality of dioptric powers at intervals of 0.25D, for example, on the eye having the wavefront aberrations normalized using the pupil diameter information. The simulation processor 150 identifies a position of the most sharply defined image or a position of the object point with the highest Strehl ratio as the dioptric power of the eye E to be examined, from among the plurality of images corresponding to the obtained dioptric powers. In the case of multifocal, the position of the most sharply defined image locally within a certain range or the object point with the highest Strehl ratio locally within a certain range is identified as the power at distant (distance) or the power at near (near objects), etc. of the eye E to be examined.

Further, the simulation processor 150 can generate a simulation image representing a view of the optotype. Examples of the optotype include Landolt rings, or images corresponding to a predetermined fixed distance. For example, the simulation processor 150 obtains a Point Spread Function (hereinafter referred to as PSF) from the wavefront aberration information normalized using the pupil diameter information using a known method, for each of the plurality of focal point distances of the IOL or one or more predetermined fixed distances. The simulation processor 150 obtains the simulation image of the fundus EF when the optotype is projected onto the fundus, by calculating the convolution integral (convolution) between the obtained PSF and the image data (brightness distribution) of the optotype.

In some embodiments, the simulation processor 150 obtains an Optical Transfer Function (hereinafter referred to as OTF) by performing Fourier transforming on the PSF. The absolute value component of the amplitude of this OTF is the Modulation Transfer Function (MTF). The phase component of the OFT is the Phase Transfer Function (PTF). The simulation processor 150 calculates the convolution of the MTF and the image data of the optotype, and also calculates the convolution of the PTF and the image data of the optotype. Furthermore, the simulation processor 150 obtains a simulation image corresponding to the convolution between the PSF and the image data of the optotype, by performing an inverse Fourier transform on the calculation results of these convolutions.

The processing performed by the simulation processor 150 like this is disclosed in, for example, Japanese Unexamined Patent Application Publication No. 2004-337236.

The controller 110 (main controller 111), as a display controller, can display the image representing the view of the eye E to be examined corresponding to the dioptric power calculated by the dioptric power calculator 130 on the display unit 170, for each focal point distance of the IOL or for each of one or more fixed distances. For example, the fixed distances include "infinity", "1 m", "40 cm", and "20 cm". The image representing the view of the eye E to be examined is a simulation image generated by the simulation processor 150 described above.

Further, the controller 110 (main control unit 111), as the display controller, can display the simulation results of the visual acuity values of the eye E to be examined on the display unit 170, for each focal point distance of the IOL or for one or more fixed distances of the IOL. The simulation results of the visual acuity values are the power (visual acuity value) of the eye E to be examined obtained by the simulation processor 150 described above.

### (Display unit 170, Operation unit 180)

The display unit 170 displays information upon receiving control of the controller 110 (main controller 111), as an interface unit. The display unit 170 includes the display unit 10 shown in FIG. 1.

The display unit 170 can receive control from the controller 110 (main controller 111) as the display controller, and can display result(s) of processing performing by the arithmetic processor 120. Examples of processing results performed by the arithmetic processor 120 include one or more dioptric powers calculated by the dioptric power calculator 130, the distribution information generated by the distribution information generator 140, and the simulation result(s) executed by the simulation processor 150.

Examples of the one or more dioptric powers calculated by the dioptric power calculator 130 include the single dioptric power calculated by the first dioptric power calculator 131, the dioptric powers, which are calculated by the second dioptric power calculator 132 or the third dioptric power calculator 133, for the number of focal points for each focal point distance (area) of the IOL, and the dioptric powers of the far point side and the near point side, which are calculated by the fourth dioptric power calculator 134. In some embodiments, the dioptric power of the far point side and the dioptric power of the near point side among the dioptric powers for the number of focal points calculated by the second dioptric power calculator 132 or the third dioptric power calculator 133 are displayed on the display unit 170. In some embodiments, a difference between the dioptric power of the farthest point side and the dioptric power of the nearest point side calculated by the fourth dioptric power calculator 134, which are calculated by the dioptric power calculator 130, is displayed on the display unit 170.

The operation unit 180 is used to operate the ophthalmic apparatus, as the user interface unit. The operation unit 180 includes various types of hardware keys (the joystick, buttons, switches, etc.) provided in the ophthalmic apparatus. Further, the operation unit 180 may include various kinds of software keys (buttons, icons, menus, etc.) displayed on the touch panel type display screen.

At least part of the display unit 170 and the operation unit 180 may be integrally configured. A typical example of this is the touch-panel display screen 10a.

### (Communication unit 190)

The communication unit 190 has the function of communicating with an external device (not shown). The communication unit 190 may be provided in the processor 9, for example. The communication unit 190 has a configuration corresponding to the mode of communication with the external device.

The arithmetic processor 120 is an example of the "ophthalmic information processing apparatus" according to the embodiments. The communication unit 190, or the aberration measurement projection system 6 and aberration measurement light reception system 7 is/are an example of the "acquisition unit" according to the embodiments. The aberration measurement projection system 6 and the aberration measurement light reception system 7 are an example of the "measurement optical system" according to the embodiments. The dioptric power calculator 130 is an example of the "calculator" according to the embodiments. The controller 110 (main controller 111) is an example of the "display controller" according to the embodiments. The display unit 170 is an example of the "display means" according to the embodiments.

### [Operation Example]

Examples of the operation of the ophthalmic apparatus according to the first embodiment will be described.

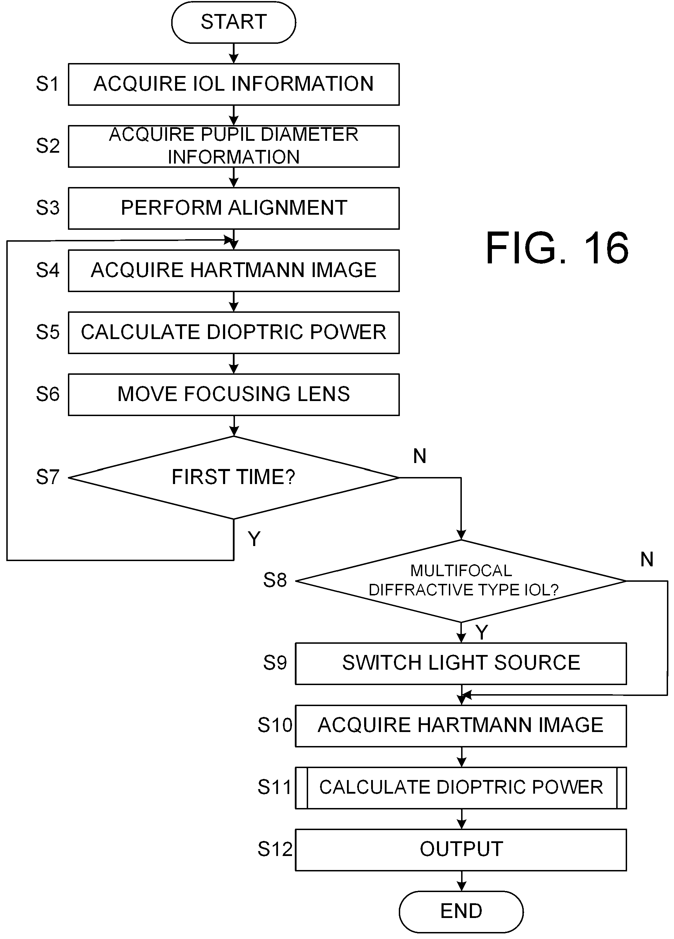

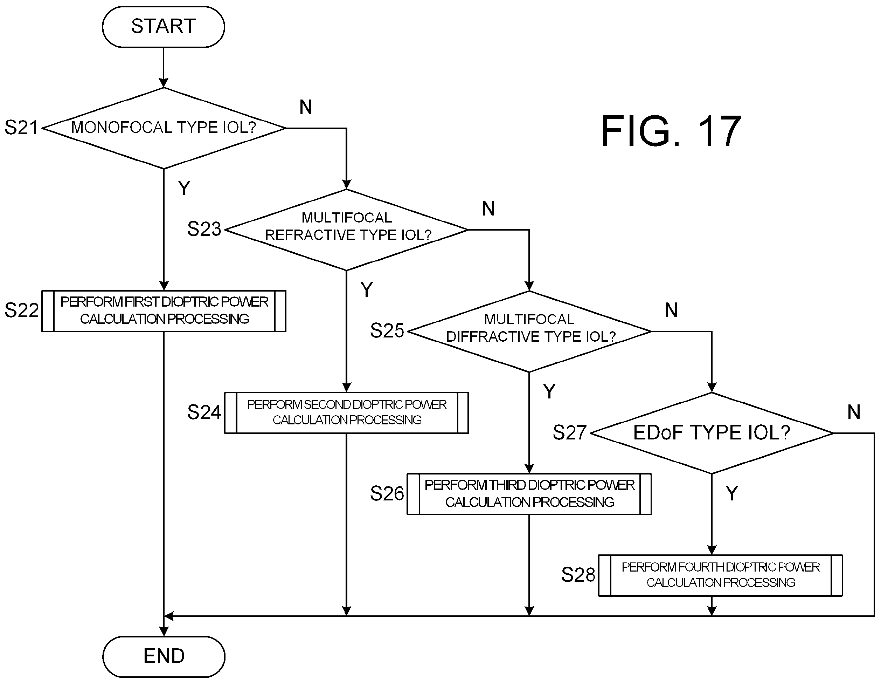

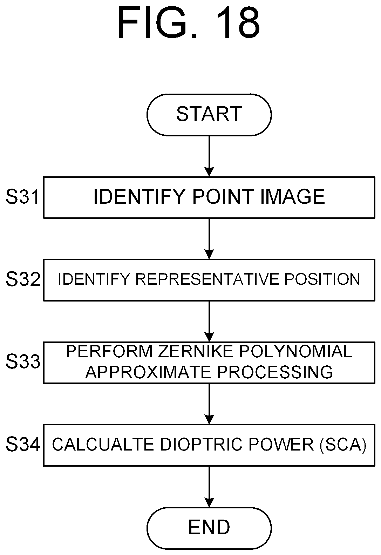

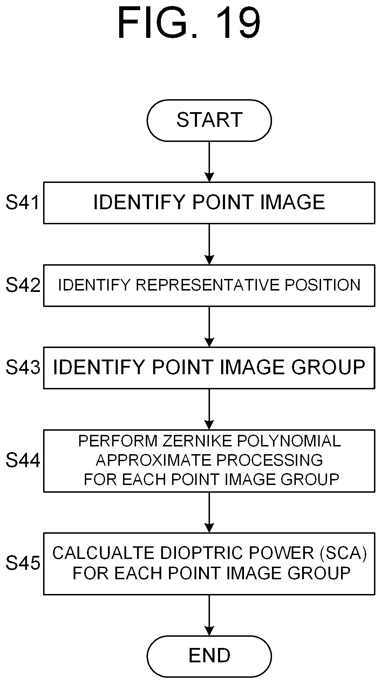

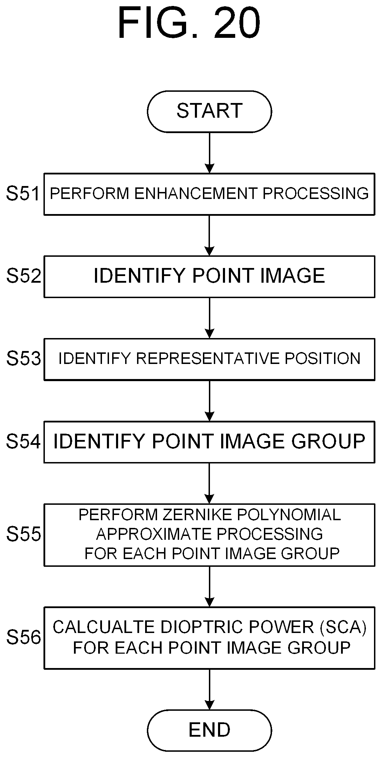

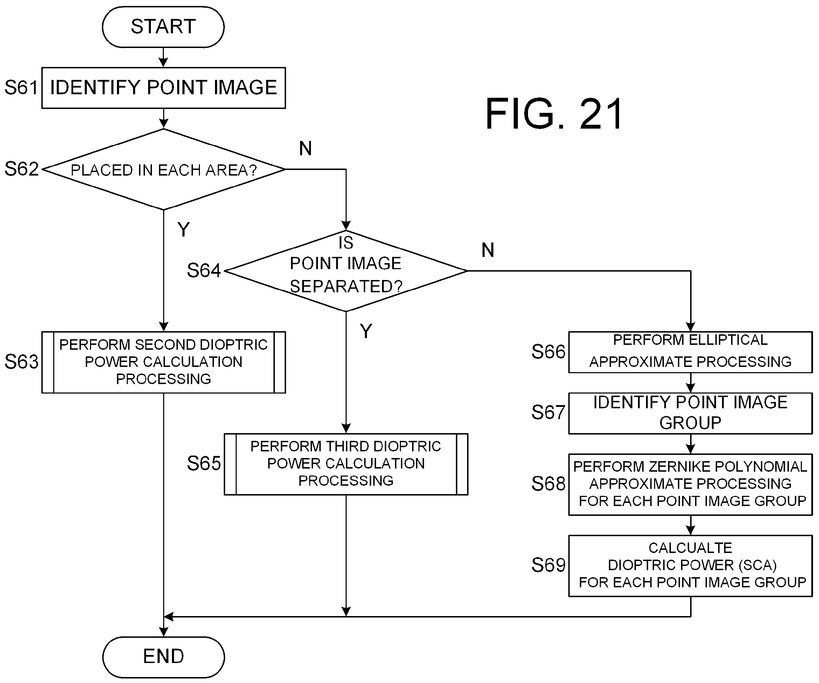

FIGS. 16 to 21 show flowcharts of examples of operations of the ophthalmic apparatus 100 according to the first embodiment. FIG. 16 represents a flowchart of an example of an operation of the ophthalmic apparatus 100 configured to calculate the dioptric power of the eye E to be examined using a calculation processing method according to the type of IOL worn by the eye E to be examined. FIG. 17 shows a flowchart of an example of the operation of step S11 in FIG. 16. FIG. 18 shows a flowchart of an example of the operation of step S22 in FIG. 17. FIG. 19 shows a flowchart of an example of the operation of step S24 in FIG. 17. FIG. 20 shows a flowchart of an example of the operation of step S26 in FIG. 17. FIG. 21 shows a flowchart of an example of the operation of step S28 in FIG. 17.

The storage unit 112 stores computer programs for realizing the processing shown in FIGS. 16 to 21. The main controller 111 operates according to the computer programs, and thereby the main controller 111 performs the processing shown in FIGS. 16 to 21.

### (S1: Acquire IOL information)

First, the main controller 111 acquires IOL information worn by the eye E to be examined.

For example, the main controller 111 controls the communication unit 190 to acquire the IOL information representing the type of the IOL worn by the eye E to be examined from the electronic health record information of the examinee, that is stored in an external device such as an ophthalmic apparatus or server connected via the communication unit 190.

In some embodiments, the main controller 111 acquires the IOL information from the type of the IOL designated based on an operation content of a user to the operation unit 180.

In some embodiments, the main controller 111 controls the observation system 5 to acquire the transillumination image or the anterior segment image of the eye E to be examined after the completion of alignment described below, and controls the arithmetic processor 120 to analyze the transillumination image or the anterior segment image to determine the type of the IOL worn by the eye E to be examined and to acquire the determined type as the IOL information. In this case, for example, the main controller 111 can acquire the transillumination image by turning one of the anterior segment illumination light sources 57 on, illuminating the fundus Ef with the illumination light from a position away from the optical axis, and receiving the returning light of the light with the area sensor 56. Alternatively, for example, the main controller 111 acquires the transillumination image by switching the XY alignment light source 21 to the SLD or the high-brightness LED, projecting the light onto the fundus Ef, and receiving the returning light of the light with the area sensor 56. Further, the main controller 111 acquires the anterior segment image of the eye E to be examined by turning the anterior segment illumination light source 27 on, and receiving the returning light with the area sensor 56. The main controller 111 can determine the type of the IOL worn by the eye E to be examined and can acquire the IOL information, using a method disclosed in Japanese Unexamined Patent Application Publication No. 2014-209994, for example.

### (S2: Acquire pupil diameter information)

Subsequently, the main controller 111 acquires the pupil diameter information represents the pupil diameter of the eye E to be examined.

For example, the main controller 111 controls the communication unit 190 to acquire the pupil diameter information of the eye E to be examined from the electronic health record information of the examinee, that is stored in the external device such as an ophthalmic apparatus or server connected via the communication unit 190.

In some embodiments, the main controller 111 acquires the pupil diameter information from the pupil diameter designated based on an operation content of the user to the operation unit 180.

In some embodiments, the main controller 111 adjusts the brightness of the light source 41 and the optotype chart 42 in the optotype projection system 4 and controls the observation system 5 to acquire the anterior segment image of the eye E to be examined after the completion of alignment described below. And then, the main controller 111 controls the arithmetic processor 120 to analyze the anterior segment image to identify the pupil diameter of the eye E to be examined and to acquire the pupil diameter information. Here, the brightness of the light source 41 and the optotype chart 42 in the optotype projection system 4 can be set, for example, closer to the daily brightness of the eye E to be examined, set to the brightness of the state desired by the eye E to be examined, or set darker to allow analysis at any pupil diameter.

### (S3: Perform alignment)

Next, the examiner performs a predetermined operation on the operation unit 180 in a state where the face of the examinee is fixed to a face supporter (not shown), and then the ophthalmic apparatus 100 starts presenting the fixation target to the eye E to be examined. Specifically, the main controller 111 controls the optotype projection system 4 to present the fixation target to the eye E to be examined.

Subsequently, the examiner performs a predetermined operation on the operation unit 180 in a state where the face of the examinee is fixed to the face supporter, and then the ophthalmic apparatus 100 performs alignment. Thereby, the head unit is moved to an inspection position for the eye E to be examined through the XY alignment performed by using the XY alignment system 2 and the Z alignment performed by using the Z alignment system 1. The inspection position is a position where the inspection of the eye E to be examined can be performed within a default accuracy.

Specifically, the main controller 111 acquires imaging signal of the anterior segment image formed on the light receiving surface of the area sensor 56 and displays the anterior segment image E' on the display unit 170 (display screen 10a of the display unit 10). After that, the head unit is moved to the inspection position for the eye E to be examined through the XY alignment and the Z alignment described above. The movement of the head unit is executed by the main controller 111 in accordance with an instruction from the main controller 111. However, the movement of the head may be executed by the main controller 111 in accordance with an operation or an instruction by the user.

After the completion of alignment, the main controller 111 moves the movement unit 69 (light source 61), the movement unit 77, and the movement unit 46 to a position of the origin (for example, a position corresponding to 0D) along the optical axis, respectively. In some embodiments, the main controller 111 moves the movement unit 69 (light source 61), the movement unit 77, and the movement unit 46 to the position of the origin (for example, the position corresponding to 0D) along the optical axis, respectively, before the completion of alignment.

### (S4: Acquire Hartmann image)

Next, the main controller 111 performs a provisional measurement.

Specifically, the main controller 111 turns on the light source 61B to irradiate near-infrared light onto the eye E to be examined in a state where the optotype is presented to the eye E to be examined using the optotype chart 42, and causes the Hartmann image (point image groups) based on the returning light from the eye E to be examined to be detected by the area sensor 76.

### (S5: Calculate dioptric power)

Next, the main controller 111 controls the dioptric power calculator 130 to calculate the dioptric power based on the intervals between the point images constituting the Hartmann image detected by the area sensor 76, and identifies a movement amount of the movement unit 77 including the collimator lens 74 as the focusing lens (equivalent to the movement amount of the focusing lens) from the calculated dioptric power. In this case, the dioptric power calculator 130 calculates the spherical power S as the dioptric power, based on the intervals between the point images that make up the Hartmann image.

### (S6: Move focusing lens)

The main controller 111 moves the movement unit 77 (collimator lens 74 as the focusing lens) to a position corresponding to the far point along the optical axis based on the movement amount corresponding to the dioptric power (spherical power S) calculated in step S5. In conjunction with this, each of the movement unit 46 and the movement unit 69 also moves in the optical axis direction by the movement amount corresponding to the dioptric power described above.

### (S7: First time?)

Subsequently, the main controller 111 determines whether or not the movement of the movement unit 77, etc. by the provisional measurement is the first time (first try).

When it is determined that the movement is the first time (S7: Y), the operation of the ophthalmic apparatus 100 proceeds to step S4. This allows for highly accurate focus control by provisional measurement.

When it is determined that the movement is not the first time (S7: N), the operation of the ophthalmic apparatus 100 proceeds to step S8.

### (S8: Multifocal diffractive type IOL?)

When it is determined in step S7 that the movement is not the first time (S7: N), the main controller 111 determines whether or not the IOL worn by the eye E to be examined is the multifocal diffractive type IOL based on the IOL information acquired in step S1.

When it is determined in step S8 that the IOL worn by the eye E to be examined is the multifocal diffractive type IOL (S8: Y), the operation of the ophthalmic apparatus 100 proceeds to step S9. When it is determined in step S8 that the IOL worn by the eye E to be examined is not the multifocal diffractive type IOL (S8: N), the operation of the ophthalmic apparatus 100 proceeds to step S10.

### (S9: Switch light source)

When it is determined in step S8 that the IOL worn by the eye E to be examined is the multifocal diffractive type (S8: Y), the main controller 111 controls the light source 61 to switch the light source for measurement from the light source 61B to the light source 61A.

### (S10: Acquire Hartmann image)

When it is determined in step S8 that the IOL worn by the eye E to be examined is not the multifocal diffractive type IOL (S8: N), the main controller 111 moves the movement unit 46 in the optical axis direction by another predetermined diopter from the position corresponding to the far point that has been moved in step S6, and causes the eye E to be examined to be promoted to the fogging of the optotype. In should be noted that the eye to be examined wearing the IOL is judged to have no amplitude of accommodation, the function for promoting to the fogging of the optotype may be omitted.

The main controller 111 turns on the light source 61B to irradiate near-infrared light onto the eye E to be examined, and causes the Hartmann image (point image groups) based on the returning light from the eye E to be examined to be detected by the area sensor 76, as the main measurement.

Alternatively, the main controller 111 turns on the light source 61A to irradiate the visible light onto the eye E to be examined, and causes the Hartmann image (point image groups) based on the returning light from the eye E to be examined to be detected by the area sensor 76, as the main measurement. Thereby, in case that the IOL worn by the eye E to be examined is the multifocal diffractive type IOL, the wavefront aberration measurement can be performed using the visible light.

### (S11: Calculate dioptric power)

Next, the main controller 111 controls the dioptric power calculator 130 to calculate the dioptric power based on the intervals between the point images constituting the Hartmann image detected by the area sensor 76. The dioptric power calculator 130 obtains the dioptric power of the eye E to be examined using a calculation processing method corresponding to the IOL information acquired in step S1. The dioptric power calculated in step S11 includes the spherical power (S), the cylindrical power (astigmatic power) (C), and the astigmatic axis angle (A). The details of step S11 will be described below.

### (S12: Output)

Subsequently, the main controller 111 displays the dioptric power calculated in step S11 on the display unit 170. In some embodiments, the main controller 111 controls the distribution information generator 140 to display the generated distribution information on the display unit 170. In some embodiments, the main controller 111 controls the simulation processor 150 to display the acquired simulation result(s) on the display unit 170. In some embodiments, the main controller 111 associates the measurement results obtained by the wavefront aberration measurement with the simulation results and displays them on the display unit 170.

This terminates the operation of the ophthalmic apparatus 100 (END).

Step S11 in FIG. 16 is performed according to the flow shown in FIG. 17.

### (S21: Monofocal type IOL?)

In step S11 of FIG. 16, the main controller 111 determines whether or not the IOL worn by the eye E to be examined is the monofocal type IOL based on the IOL information acquired in step S1.

When it is determined in step S21 that the IOL worn by the eye E to be examined is the monofocal type IOL (S21: Y), the operation of the ophthalmic apparatus 100 proceeds to step S22. When it is determined in step S21 that the IOL worn by the eye E to be examined is not the monofocal type IOL (S21: N), the operation of the ophthalmic apparatus 100 proceeds to step S23.

### (S22: Perform first dioptric power calculation processing)

When it is determined in step S21 that the IOL worn by the eye E to be examined is the monofocal type IOL (S21: Y), the main controller 111 controls the first dioptric power calculator 131 to perform the first dioptric power calculation processing, in which the dioptric power of the eye E to be examined is calculated using a calculation processing method corresponding to the monofocal type IOL, based on the wavefront aberration information acquired in step S10. The details of step S22 will be described below.

This terminates the processing of step S11 in FIG. 16 (END).

### (S23: Multifocal refractive type IOL?)

When it is determined in step S21 that the IOL worn by the eye E to be examined is not the monofocal type IOL (S21: N), the main controller 111 determines whether or not the IOL worn by the eye E to be examined is the multifocal refractive type IOL based on the IOL information acquired in step S1.

When it is determined in step S23 that the IOL worn by the eye E to be examined is the multifocal refractive type IOL (S23: Y), the operation of the ophthalmic apparatus 100 proceeds to step S24. When it is determined in step S23 that the IOL worn by the eye E to be examined is not the multifocal refractive type IOL (S23: N), the operation of the ophthalmic apparatus 100 proceeds to step S25.

### (S24: Perform second dioptric power calculation processing)

When it is determined in step S23 that the IOL worn by the eye E to be examined is the multifocal refractive type IOL (S23: Y), the main controller 111 controls the second dioptric power calculator 132 to perform the second dioptric power calculation processing, in which the dioptric power of the eye E to be examined is calculated using a calculation processing method corresponding to the multifocal refractive type IOL, based on the wavefront aberration information acquired in step S10. The details of step S24 will be described below.

This terminates the processing of step S11 in FIG. 16 (END).

### (S25: Multifocal diffractive type IOL?)

When it is determined in step S23 that the IOL worn by the eye E to be examined is not the multifocal refractive type IOL (S23: N), the main controller 111 determines whether or not the IOL worn by the eye E to be examined is the multifocal diffractive type IOL based on the IOL information acquired in step S1.

When it is determined in step S25 that the IOL worn by the eye E to be examined is the multifocal diffractive type IOL (S25: Y), the operation of the ophthalmic apparatus 100 proceeds to step S26. When it is determined in step S25 that the IOL worn by the eye E to be examined is not the multifocal diffractive type IOL (S25: N), the operation of the ophthalmic apparatus 100 proceeds to step S27.

### (S26: Perform third dioptric power calculation processing)

When it is determined in step S25 that the IOL worn by the eye E to be examined is the multifocal diffractive type IOL (S25: Y), the main controller 111 controls the third dioptric power calculator 133 to perform the third dioptric power calculation processing, in which the dioptric power of the eye E to be examined is calculated using a calculation processing method corresponding to the multifocal diffractive type IOL, based on the wavefront aberration information acquired in step S10. The details of step S26 will be described below.

This terminates the processing of step S11 in FIG. 16 (END).

### (S27: EDoF type IOL?)

When it is determined in step S25 that the IOL worn by the eye E to be examined is not the multifocal diffractive type IOL (S25: N), the main controller 111 determines whether or not the IOL worn by the eye E to be examined is the EDoF type IOL based on the IOL information acquired in step S1.

When it is determined in step S27 that the IOL worn by the eye E to be examined is the EDoF type IOL (S27: Y), the operation of the ophthalmic apparatus 100 proceeds to step S28. When it is determined in step S27 that the IOL worn by the eye E to be examined is not an EDoF type IOL (S27: N), the operation of the ophthalmic apparatus 100 is terminated (END).

### (S28: Perform fourth dioptric power calculation processing)

When it is determined in step S27 that the IOL worn by the eye E to be examined is the EDoF type IOL (S27: Y), the main controller 111 controls the fourth dioptric power calculator 134 to perform the fourth dioptric power calculation processing, in which the dioptric power of the eye E to be examined is calculated using a calculation processing method corresponding to the EDoF type IOL, based on the wavefront aberration information acquired in step S10. The details of step S28 will be described below.

Subsequent to step S28, the processing of step S11 in FIG. 16 is terminated (END).

In step S22 of FIG. 17, the first dioptric power calculation processing is performed by the first dioptric power calculator 131 according to the flow shown in FIG. 18.

### (S31: Identify point image)

In step S22 of FIG. 17, first, the main controller 111 controls the point image identifying unit 131A to identify the point images constituting the Hartmann image acquired in step S10.

### (S32: Identify representative position)

Next, the main controller 111 controls the representative position identifying unit 131B to identify the representative position (in this case, position of the center of gravity) of the point image identified in step S31.

### (S33: Perform Zernike polynomial approximate processing)

Subsequently, the main controller 111 controls the Zernike polynomial approximate processor 131C to calculate the wavefront aberration information (approximate expression for wavefront) by performing Zernike polynomial approximate processing based on the representative position(s) of the plurality of point images identified in step S32 and the pupil diameter information acquired in step S2. Here, the wavefront aberration information is represented by the Zernike coefficient(s) and the Zernike polynomial. The Zernike polynomial approximate processor 131C normalizes the calculated wavefront aberration information using the pupil diameter information acquired in step S2.

### (S34: Calculate dioptric power (SCA))

Next, the main controller 111 controls the first dioptric power calculator 131 to obtain the spherical power S, the astigmatic power C, and the astigmatic axis angle A from the Zernike coefficients obtained by the Zernike polynomial approximate processing performed in step S33, using a known operation.

This terminates the first dioptric power calculation processing of step S22 in FIG. 17 (END).

In step S24 of FIG. 17, the second dioptric power calculation processing is performed by the second dioptric power calculator 132 according to the flow shown in FIG. 19.

### (S41: Identify point image)

In step S24 of FIG. 17, first, the main controller 111 controls the point image identifying unit 132A to identify the point images constituting the Hartmann image acquired in step S10. In some embodiments, the main controller 111 controls the second dioptric power calculator 132 to perform enhancement processing of point image on the Hartmann image acquired in step S10, and then controls the point image identifying unit 132A as described above.

### (S42: Identify representative position)

Next, the main controller 111 controls the representative position identifying unit 132B to identify the representative position (in this case, position of the center of gravity) of the point image identified in step S41.

### (S43: Identify point image group)

Subsequently, the main controller 111 controls the point image group identifying unit 132C to identify which area among a plurality of areas predetermined for each focal point distance of the IOL includes the point image constituting the Hartmann image based on the representative position identified in step S42. Here, if the position of the IOL within the pupil can be identified, the positional relationship indicating where each of the plurality of areas for each focal point distance of the IOL (areas for the number of focal points) are placed in the Hartmann image is known. Therefore, the point image group identifying unit 132C can identify in which area the representative position of the point images is placed based on the type of the IOL identified by the IOL information. The point image group identifying unit 132C classifies the point images by focal point distance (area) of the IOL (see FIG. 10 and FIG. 11). The point image group identifying unit 132C may identify the point image group by identifying an area where the intervals between the point images are narrowing with reference to a predetermined reference interval and an area where the intervals between the point images are widening with reference to a predetermined reference interval.

For example, when the number of the focal points of the IOL worn by the eye E to be examined is "2", the point image group identifying unit 132C identifies a point image group including one or more point images belonging to the near area(s) and a point image group including one or more point images belonging to the far area(s). For example, when the number of the focal points of the IOL worn by the eye E to be examined is "3", the point image group identifying unit 132C identifies a point image group including one or more point images belonging to the near area(s), a point image group including one or more point images belonging to one or more medium area(s), and a point image group including one or more point images belonging to the far area(s). For example, when the number of the focal points of the IOL worn by the eye E to be examined is 4 or more, the point image group identifying unit 132C identifies a point image group including one or more point images belonging to the near area(s), a point image group including one or more point images belonging to two or more medium area(s), and a point image group including one or more point images belonging to the far area(s).

### (S44: Perform Zernike polynomial approximate processing for each point image group)

Subsequently, the main controller 111 controls the Zernike polynomial approximate processor 132D to calculate the wavefront aberration information (approximate expression for wavefront), that is represented by the Zernike coefficient(s) and the Zernike polynomial, for each point image group by performing Zernike polynomial approximate processing for each point image group identified in step S43. The Zernike polynomial approximate processor 132D normalizes each of the calculated wavefront aberration information calculated for each point image group, using the pupil diameter information acquired in step S2.

### (S45: Calculate dioptric power (SCA) for each point image group)

Next, the main controller 111 controls the second dioptric power calculator 132 to obtain the spherical power S, the astigmatic power C, and the astigmatic axis angle A for each point image group from the Zernike coefficients obtained by the Zernike polynomial approximate processing performed for each point image group in step S44, using a known operation.

This terminates the second dioptric power calculation processing of step S24 in FIG. 17 (END).

In step S26 of FIG. 17, the third dioptric power calculation processing is performed by the third dioptric power calculator 133 according to the flow shown in FIG. 20.

### (S51: Perform enhancement processing)

In step S26 of FIG. 17, first, the main controller 111 controls the enhancement processor 133A to perform enhancement processing of the point image (separated point image) on the Hartmann image acquired in step S10. Thereby, the two or more separated point images (in particular, separated point image on the near point side), that are formed by separating the point image constituting the Hartmann image, can be easily identified.

### (S52: Identify point image)

Next, the main controller 111 controls the point image identifying unit 133B to identify the separated point image constituting the Hartmann image on which the enhancement processing of the separated point image has been performed in step S51.

### (S53: Identify representative position)

Next, the main controller 111 controls the representative position identifying unit 133C to identify the representative position (in this case, position of the center of gravity) of the separated point image identified in step S51.

### (S54: Identify point image group)

Subsequently, the main controller 111 controls the point image group identifying unit 133D to identify point image group for each focal point distance of the IOL, for the separated point image, based on the representative position identified in step S53. The point image group identifying unit 132C classifies the separated point images identified in step S52 into any one of the two or more point image groups determined according to the type of the IOL identified by the IOL information.

For example, when the number of the focal points of the IOL worn by the eye E to be examined is "2", the point image group identifying unit 133D identifies the point image group of the separated point images at the near point and the point image group of the separated point images at the far point, by classifying each of the plurality of separated point images identified in step S52 into either the point image group of the separated point images at the near point or the point image group of the separated point images at the far point. For example, when the number of the focal points of the IOL worn by the eye E to be examined is "3", the point image group identifying unit 133D identifies the point image group of the separated point images at the near point, the point image group of the separated point images at the medium point, and the point image group of the separated point images at the far point, by classifying each of the plurality of separated point images identified in step S52 into any one of the point image group of the separated point images at the near point, the point image group of the separated point images at the medium point, and the point image group of the separated point images at the far point. For example, when the number of the focal points of the IOL worn by the eye E to be examined is 4 or more, the point image group identifying unit 133D identifies the point image group of the separated point images at the near point, the two or more point image groups of the separated point images at the two or more medium points, and the point image group of the separated point images at the far point, by classifying each of the plurality of separated point images identified in step S52 into either the point image group of the separated point images at the near point, the two or more point image group of the separated point images at the two or more medium points, or the point image group of the separated point images at the far point.

### (S55: Perform Zernike polynomial approximate processing for each point image group)

Subsequently, the main controller 111 controls the Zernike polynomial approximate processor 133E to calculate the wavefront aberration information (approximate expression for wavefront), that is represented by the Zernike coefficient(s) and the Zernike polynomial, for each point image group by performing Zernike polynomial approximate processing for each point image group identified in step S54. The Zernike polynomial approximate processor 133E normalizes each of the calculated wavefront aberration information calculated for each point image group, using the pupil diameter information acquired in step S2.

### (S56: Calculate dioptric power (SCA) for each point image group)

Next, the main controller 111 controls the third dioptric power calculator 133 to obtain the spherical power S, the astigmatic power C, and the astigmatic axis angle A for each point image group from the Zernike coefficients obtained by the Zernike polynomial approximate processing performed for each point image group in step S55, using a known operation.

This terminates the third dioptric power calculation processing of step S26 in FIG. 17 (END).

In step S28 of FIG. 17, the fourth dioptric power calculation processing is performed by the fourth dioptric power calculator 134 according to the flow shown in FIG. 21.

### (S61: Identify point image)

In step S28 of FIG. 17, first, the main controller 111 controls the point image identifying unit 134A to identify the point images constituting the Hartmann image acquired in step S10. In some embodiments, the main controller 111 controls the fourth dioptric power calculator 134 to perform enhancement processing of point image on the Hartmann image acquired in step S10, and then controls the point image identifying unit 134A as described above.

### (S62: Placed in each area?)

Next, the main controller 111 determines whether or not the plurality of the point images identified in step S61 is placed in each area corresponding to the focal point distance of the IOL, as shown in FIG. 10 or FIG. 11. In some embodiments, the main controller 111 controls the fourth dioptric power calculator 134 to identify the representative position of the point image identified in step S61 in the same way as in step S42, and determines whether or not the representative position is placed in each area corresponding to the focal point distance of the IOL, based on the identified representative position in the same way as in step S43.

When it is determined that the plurality of identified point images is placed in each area corresponding to the focal point distance of the IOL (S62: Y), the fourth dioptric power calculation processing proceeds to step S63. When it is determined that the plurality of identified point images is not placed in each area corresponding to the focal point distance of the IOL (S62: N), the fourth dioptric power calculation processing proceeds to step S64.

### (S63: Perform second dioptric power calculation processing)

When it is determined in step S62 that the plurality of identified point images is placed in each area corresponding to the focal point distance of the IOL (S62: Y), the main controller 111 controls the second dioptric power calculator 132 to perform the second dioptric power calculation processing, in which the dioptric power of the eye E to be examined is calculated, based on the point image(s) identified in step S61. The second dioptric power calculator 132 performs the second dioptric power calculation processing according to the flow shown in FIG. 19. This terminates the processing of step S28 in FIG. 17 (END).

### (S64: Is point image separated?)

When it is determined in step S62 that the plurality of identified point images are not placed in each area corresponding to the focal point distance of the IOL (S62: N), the main controller 111 determines whether or not the point images, the number of which is equal to or greater than a predetermined threshold value among the point images constituting the Hartmann image, are separated into the two or more separated point images as shown in FIG. 13, for the point images identified in step S61.

When it is determined that the point images, the number of which is equal to or greater than a predetermined threshold value among the identified point images, are separated into the two or more separated point images (S64: Y), the fourth dioptric power calculation processing proceeds to step S65. When it is determined that the point images, the number of which is equal to or greater than a predetermined threshold value among the identified point images, are not separated into the two or more separated point images (S64: N), the fourth dioptric power calculation processing proceeds to step S66.

### (S65: Perform third dioptric power calculation processing)

When it is determined that the point images, the number of which is equal to or greater than a predetermined threshold value among the identified point images, are separated into the two or more separated point images (S64: Y), the main controller 111 controls the third dioptric power calculator 133 to perform the third dioptric power calculation processing, in which the dioptric power of the eye E to be examined is calculated, based on the point image(s) identified in step S61. The third dioptric power calculator 133 performs the third dioptric power calculation processing according to the flow shown in FIG. 20. This terminates the processing of step S28 in FIG. 17 (END).

### (S66: Perform elliptical approximate processing)

When it is determined in step S64 that the point images, the number of which is equal to or greater than a predetermined threshold value among the identified point images, are not separated into the two or more separated point images (S64: N), the main controller 111 controls the elliptical approximate processor 134B to perform known elliptical approximate processing on each of the point images identified in step S61. The elliptical approximate processor 134B identifies a plurality of approximate ellipses of the point images identified in step S61, and identifies the two focal points of each of the identified approximate ellipses.

### (S67: Identify point image group)

Next, the main controller 111 controls the point image group identifying unit 134C to identify the focal point closer to the center of the Hartmann image among the two focal points of the approximate ellipse identified in step S66 as the focal point at near point, and the focal point farther from the center of the Hartmann image among the two focal points of the approximate ellipse identified in step S66 as the focal point at far point. The point image group identifying unit 134C classifies the plurality of point images at near point and the plurality of point images at far point, which are identified for each of the plurality of approximate ellipses, into a point image group of the point image at near point and a point image group of the point image at far point.

### (S68: Perform Zernike polynomial approximate processing for each point image group)

Subsequently, the main controller 111 controls the Zernike polynomial approximate processor 134D to calculate the wavefront aberration information (approximate expression for wavefront), that is represented by the Zernike coefficient(s) and the Zernike polynomial, for each point image group by performing Zernike polynomial approximate processing for each point image group identified in step S67. The Zernike polynomial approximate processor 134D normalizes each of the calculated wavefront aberration information calculated for each point image group, using the pupil diameter information acquired in step S2.

### (S69: Calculate dioptric power (SCA) for each point image group)

Next, the main controller 111 controls the fourth dioptric power calculator 134 to obtain the spherical power S, the astigmatic power C, and the astigmatic axis angle A for each point image group from the Zernike coefficients obtained by the Zernike polynomial approximate processing performed for each point image group in step S68, using a known operation.

This terminates the fourth dioptric power calculation processing of step S28 in FIG. 17 (END).

In step S12 of FIG. 16, for example, the main controller 111 can display the following information on the display unit 170.

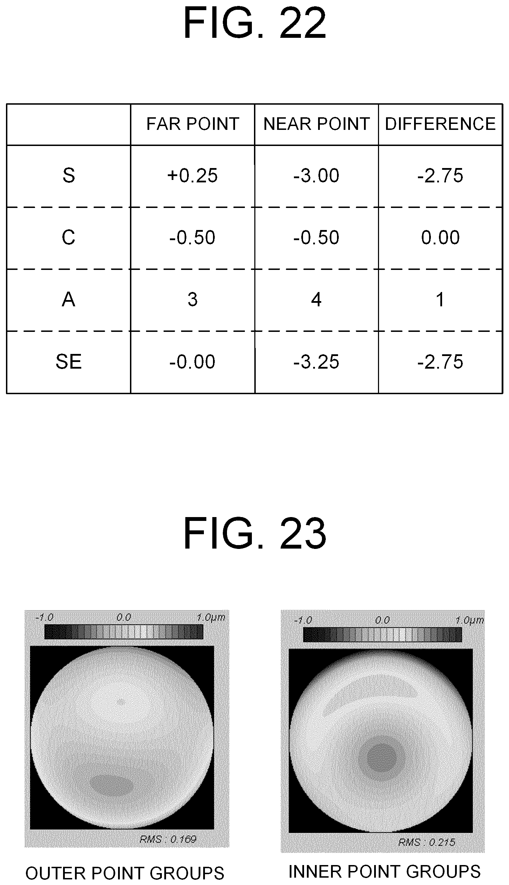

FIG. 22 shows a first display example of the display unit 170 in step S12 of FIG. 16. The first display example is a display example of the calculation results of the dioptric power.

The main controller 111 controls the dioptric power calculator 130 to calculate an equivalent spherical power (SE) using a known calculation method from the dioptric power (SCA) calculated in step S11 of FIG. 16. Thereby, the dioptric power (spherical power (S), cylindrical power (C), astigmatic axis angle (A), and equivalent spherical power (SE)) is calculated for each focal point distance of the IOL, in accordance with the type of IOL, except for the monofocal type IOL. The main controller 111 displays the calculated dioptric power on the display unit 170.

The dioptric power calculator 130 can calculate a difference between the far point and the near point, for each of spherical power (S), cylinder power (C), astigmatic axis angle (A), and equivalent spherical power (SE).

For example, the main controller 111 displays the dioptric power at the far point and the dioptric power at the near point on the display unit 170 from among the dioptric powers calculated for each focal point distance (area) of the IOL according to the type of IOL (FIG. 22).

FIG. 23 shows a second display example of the display unit 170 in step S12 of FIG. 16. The second display example is a display example of the distribution information.

The main controller 111 controls the distribution information generator 140 to generate the distribution information (wavefront aberration map) representing the distribution of the wavefront aberrations, for each focal point distance of the IOL. The main controller 111 displays the generated distribution information on the display unit 170.

The main controller 111 can generate the distribution information of the far point and the near point among the distribution information that can be generated for each focal point distance of the IOL, and can display the generated distribution information of the outer point groups (point groups at far point) and the inner point groups (point groups at near point) on the display unit 170 (FIG. 23).

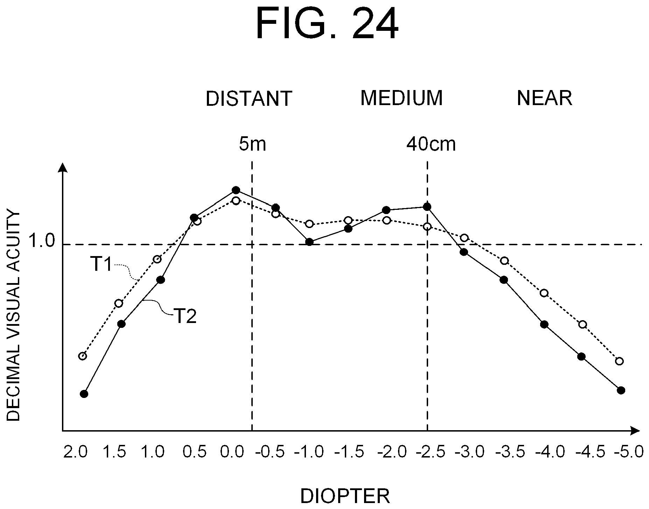

FIG. 24 shows a third display example of the display unit 170 in step S12 of FIG. 16. The third display example is a display example of the simulation result.

The main controller 111 controls the simulation processor 150 to perform visual acuity simulation. The main controller 111 displays the generated simulation result(s) on the display unit 170. The simulation processor 150 obtains a plurality of visual acuity values of the eye E to be examined corresponding to each of a plurality of object points, while changing the position of the object point.

The main controller 111 can display the visual acuity simulation result T2 on the display unit 170, superimposed on the characteristic information (product specifications) T1 of the IOL identified based on the IOL information (FIG. 24).

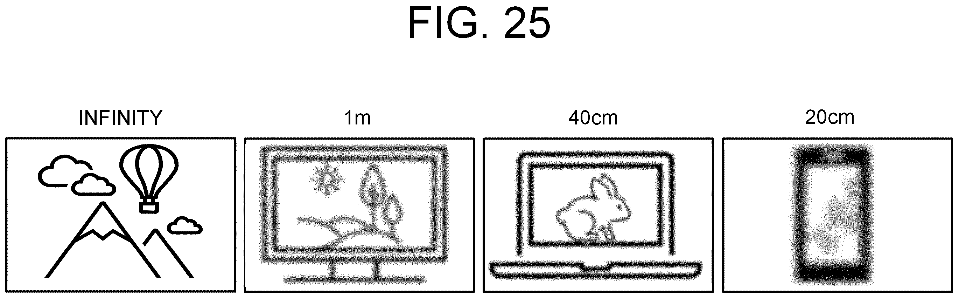

FIG. 25 shows a fourth display example of the display unit 170 in step S12 of FIG. 16. The fourth display example is a display example of the simulation image representing the view of the optotype.

The main controller 111 controls the simulation processor 150 to generate the simulation image of the fundus Ef when the optotype is projected onto the fundus Ef, by obtaining PSF, and calculating the convolution integral (convolution) between the obtained PSF and the image data (brightness distribution) of the optotype, for each of the plurality of focal point distances of the IOL or predetermined one or more fixed distances.

In FIG. 25, the images of the optotype corresponding to fixed distances include an image of a landscape corresponding to the optotype at an infinity, an image representing a desktop personal computer screen corresponding to the optotype at "1 m", an image representing a laptop computer screen corresponding to the optotype at "40 cm", and an image representing a smartphone screen corresponding to the optotype at "20 cm". The simulation processor 150 calculates the convolution integral between the image data of each image shown in FIG. 25 and PSF. The main controller 111 displays the simulation images representing the view of the optotypes at fixed distances of "infinity", "1 m", "40 cm", and "20 cm" on the display unit 170. It should be noted that the optotype may be a Landolt ring in FIG. 25.

As described above, according to the first embodiment, the dioptric power of the eye to be examined wearing the IOL is calculated using a calculation processing method in accordance with the type of the IOL worn by the eye to be examined from the wavefront aberration information acquired in a focused state with respect to the eye to be examined, corresponding to an average focal point distance of the IOL. Thereby, the reliability of the calculation result of the dioptric power of the eye to be examined wearing an IOL can be improved.

### <Second embodiment>

Generally, it is thought that the eye wearing the IOL will lose the function of accommodation. However, there is a possibility that the function of accommodation can be restored to the eye to be examined wearing the IOL through the movement of the ciliary muscle, etc. Therefore, in the second embodiment, the wavefront aberration measurement is performed at two or more distances including the far point side and the near point side, and a distant power (dioptric power at far point) and a near (reading) power (dioptric power at near point) are calculated at each position.

Hereinafter, the configuration and the operation of the ophthalmic apparatus according to the second embodiment will be described, focusing on the differences from the ophthalmic apparatus 100 according to the first embodiment.

The ophthalmic apparatus according to the second embodiment is almost the same configuration as the ophthalmic apparatus 100 according to the first embodiment.

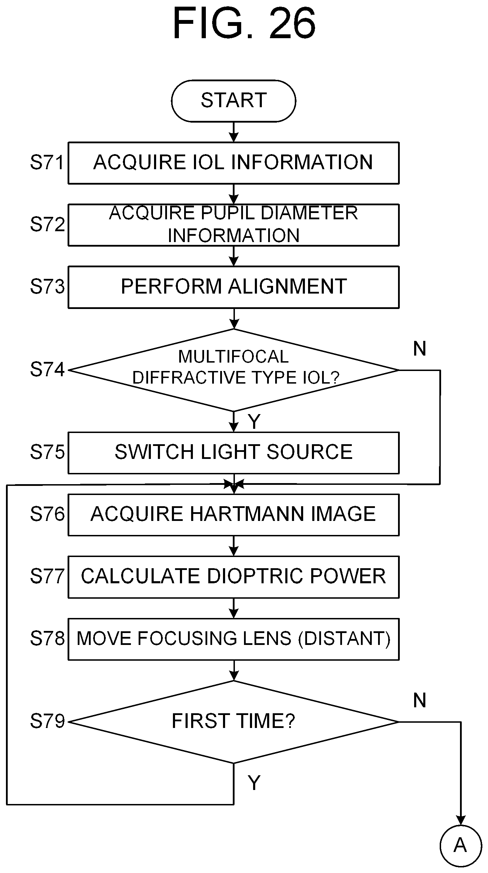

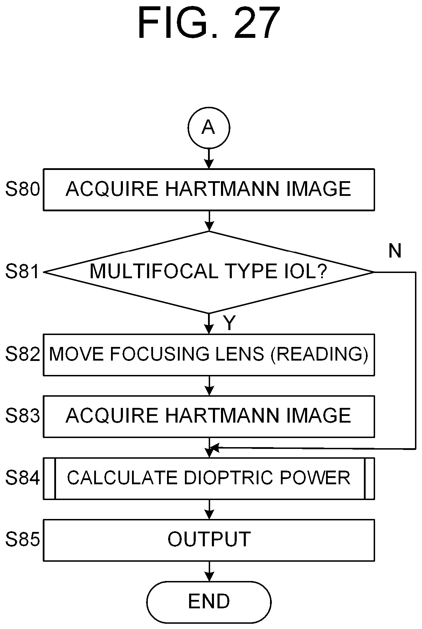

FIG. 26 and FIG. 27 show flowcharts of examples of the operation of the ophthalmic apparatus according to the second embodiment. FIG. 26 and FIG. 27 represent flowcharts of examples of the operation of the ophthalmic apparatus configured to calculate the dioptric power of the eye E to be examined using a calculation processing method according to the type of IOL worn by the eye E to be examined. The storage unit 112 stores computer program(s) for realizing the processing shown in FIG. 26 and FIG. 27. The main controller 111 operates according to the computer program(s), and thereby the main controller 111 performs the processing shown in FIG. 26 and FIG. 27.

### (S71: Acquire IOL information)

First, the main controller 111 acquires IOL information worn by the eye E to be examined, in the same way as in step S1.

### (S72: Acquire pupil diameter information)

Subsequently, the main controller 111 acquires the pupil diameter information represents the pupil diameter of the eye E to be examined, in the same way as in step S2.

### (S73: Perform alignment)

Next, the main controller 111 performs alignment in a state where the fixation target is presented to the eye E to be examined, in the same way as in step S3.

After the completion of alignment, the main controller 111 moves the movement unit 69 (light source 61), the movement unit 77, and the movement unit 46 to a position of the origin (for example, a position corresponding to 0D) along the optical axis, respectively. In some embodiments, the main controller 111 moves the movement unit 69 (light source 61), the movement unit 77, and the movement unit 46 to the position of the origin (for example, the position corresponding to 0D) along the optical axis, respectively, before the completion of alignment.

### (S74: Multifocal diffractive type IOL?)

Next, the main controller 111 determines whether or not the IOL worn by the eye E to be examined is the multifocal diffractive type IOL based on the IOL information acquired in step S71, in the same way as in step S8.

When it is determined in step S74 that the IOL worn by the eye E to be examined is the multifocal diffractive type IOL (S74: Y), the operation of the ophthalmic apparatus proceeds to step S75. When it is determined in step S74 that the IOL worn by the eye E to be examined is not the multifocal diffractive type IOL (S74: N), the operation of the ophthalmic apparatus proceeds to step S76.

### (S75: Switch light source)

When it is determined in step S74 that the IOL worn by the eye E to be examined is the multifocal diffractive type (S74: Y), the main controller 111 controls the light source 61 to switch the light source for measurement from the light source 61B to the light source 61A, in the same way as in step S9.

### (S76: Acquire Hartmann image)

Subsequent to step S75, or when it is determined in step S74 that the IOL worn by the eye E to be examined is not the multifocal diffractive type IOL (S74: N), the main controller 111 perform provisional measurement, in the same way as in step S4.

### (S77: Calculate dioptric power)

Next, the main controller 111 controls the dioptric power calculator 130 to calculate the dioptric power based on the intervals between the point images constituting the Hartmann image detected by the area sensor 76, and identifies a movement amount of the movement unit 77 including the collimator lens 74 as the focusing lens from the calculated dioptric power, in the same way as in step S5.

### (S78: Move focusing lens (distant))

The main controller 111 moves the movement unit 77 (collimator lens 74 as the focusing lens) to a position corresponding to the far point along the optical axis based on the movement amount corresponding to the dioptric power (spherical power S) calculated in step S77, in the same way as in step S6. In conjunction with this, each of the movement unit 46 and the movement unit 69 also moves in the optical axis direction by the movement amount corresponding to the dioptric power described above.

### (S79: First time?)

Subsequently, the main controller 111 determines whether or not the movement of the movement unit 77, etc. by the provisional measurement is the first time (first try), in the same way as in step S7.

When it is determined that the movement is the first time (S79: Y), the operation of the ophthalmic apparatus proceeds to step S76. When it is determined that the movement is not the first one (S79: N), the operation of the ophthalmic apparatus proceeds to step S80.

### (S80: Acquire Hartmann image)

When it is determined in step S79 that the IOL worn by the eye E to be examined is not the first time (S79: N), the main controller 111 moves the movement unit 46 in the optical axis direction by another predetermined diopter from the position corresponding to the far point that has been moved in step S78, and causes the eye E to be examined to be promoted to the fogging of the optotype. In should be noted that the eye to be examined wearing the IOL is judged to have no amplitude of accommodation, the function for promoting to the fogging of the optotype may be omitted.

The main controller 111 irradiates the light (near-infrared light or visible light) from the light source 61 (light source 61A or light source 61B) onto the eye E to be examined, and causes the Hartmann image (point image groups) based on the returning light from the eye E to be examined to be detected by the area sensor 76, as the main measurement.

### (S81: Multifocal type IOL?)

Subsequently, the main controller 111 determines whether or not the IOL worn by the eye E to be examined is the multifocal type IOL based on the IOL information acquired in step S71.

When it is determined that the IOL worn by the eye E to be examined is the multifocal type IOL (S81: Y), the operation of the ophthalmic apparatus proceeds to step S82. When it is determined that the IOL worn by the eye E to be examined is not the multifocal type IOL (S81: N), the operation of the ophthalmic apparatus proceeds to step S84.

### (S82: Move focusing lens (reading))

When it is determined in step S81, that the IOL worn by the eye E to be examined is the multifocal type IOL (S81: Y), the main controller 111 moves the movement unit 77 (collimator lens 74 as the focusing lens) to a reading position along the optical axis. In conjunction with this, each of the movement unit 46 and the movement unit 69 also moves in the optical axis direction by the movement amount corresponding to the dioptric power described above. Examples of the reading position include a fixed position such as 40 cm, a position frequently used by the eye E to be examined, and a predetermined reading position corresponding to the IOL worn by the eye E to be examined.

### (S83: Acquire Hartmann image)

Next, the main controller 111 irradiates the light (near-infrared light or visible light) from the light source 61 (light source 61A or light source 61B) onto the eye E to be examined, and causes the Hartmann image (point image groups) based on the returning light from the eye E to be examined to be detected by the area sensor 76, as the main measurement.

### (S84: Calculate dioptric power)

Subsequent to step S83, or when it is determined in step S81 that the IOL worn by the eye E to be examined is not the multifocal type IOL (S81: N), the main controller 111 controls the dioptric power calculator 130 to calculate the dioptric power based on the intervals of the point images constituting the Hartmann image detected by the area sensor 76 in step S83 or step S76, in the same way as in step S11. The dioptric power calculator 130 obtains the dioptric power of the eye E to be examined using a calculation processing method corresponding to the IOL information acquired in step S71, in the same way as in step S11.

### (S85: Output)

Subsequently, the main controller 111 displays the dioptric power calculated in step S84 on the display unit 170, in the same way as in step S12. In some embodiments, the main controller 111 controls the distribution information generator 140 to display the generated distribution information on the display unit 170. In some embodiments, the main controller 111 controls the simulation processor 150 to display the acquired simulation result(s) on the display unit 170. In some embodiments, the main controller 111 associates the measurement results obtained by the wavefront aberration measurement with the simulation results and displays them on the display unit 170.

This terminates the operation of the ophthalmic apparatus according to the second embodiment (END).

For example, the distant power and the reading power can be obtained from the Hartmann image acquired by performing distant measurement in step S76, and the distant power and the reading power can be obtained from the Hartmann image acquired by performing reading measurement in step S83. In this case, the ophthalmic apparatus may select and output the distant power acquired by performing the distant measurement and the reading power acquired by performing the reading measurement.

It should be noted that the case where the number of focal points of the IOL is "1" (monofocal type) or "2" has been described in FIG. 26 and FIG. 27. However, the second embodiment is not limited to this case. For example, when the number of focal points is "3" or more, it is possible to repeat step S82 to step S83 to acquire Hartmann images for the number of focal points and to calculate the dioptric powers for the number of focal points for each of the acquired Hartmann images.

As described above, according to the second embodiment, the plurality of Hartmann images, each of which is acquired in a state of being focused on the eye E to be examined corresponding to each of the plurality of focal point distances of the IOL, and the dioptric power is calculated using each of the Hartmann images. Thereby, even when the eye E to be examined wearing the IOL has the function of accommodation, the reliability of the calculation results of the dioptric power of the eye E to be examined wearing the IOL can be improved.

### [Actions]

The ophthalmic information processing apparatus, the ophthalmic apparatus, the ophthalmic information processing method, and the program according to the embodiments will be described.

The first aspect of some embodiments is an ophthalmic information processing apparatus (arithmetic processor 120) configured to calculate a dioptric power of an eye (E) to be examined based on wavefront aberration information obtained by performing wavefront aberration measurement on the eye to be examined wearing an intraocular lens. The ophthalmic information processing apparatus includes an acquisition unit (communication unit 190, or aberration measurement projection system 6 and aberration measurement light reception system 7), and a calculator (dioptric power calculator 130). The acquisition unit is configured to acquire intraocular lens information representing at least optical characteristics of the intraocular lens. The calculator is configured to change calculation processing method of the dioptric power in accordance with the intraocular lens information to calculate the dioptric power of the eye to be examined.

According to such an aspect, the dioptric power of the eye to be examined wearing the IOL is calculated using a calculation processing method in accordance with the type of the IOL worn by the eye to be examined. Thereby, the reliability of the calculation result of the dioptric power of the eye to be examined wearing an IOL can be improved.

In the ophthalmic information processing apparatus according to the second aspect of some embodiments, in the first aspect, the acquisition unit is configured to acquire pupil diameter information representing a pupil diameter. The calculator is configured to calculate the dioptric power based on wavefront aberration information within a region demarcated based on the pupil diameter information.

According to such an aspect, the dioptric power is calculated based on the wavefront aberration information corresponding to the pupil diameter. Thereby, the reliability of the calculation result of the dioptric power of the eye to be examined wearing an IOL can be further improved.

In the ophthalmic information processing apparatus according to the third aspect of some embodiments, the wavefront aberration information includes a Hartmann image acquired in a focused state with respect to the eye to be examined, corresponding to an average focal point distance of the intraocular lens.

According to such an aspect, the reliability of the calculation result of the dioptric power of the eye to be examined wearing the IOL can be improved, with simple controls, depending on the type of IOL.

In the ophthalmic information processing apparatus according to the fourth aspect of some embodiments, in the first aspect, the wavefront aberration information includes a plurality of Hartmann images acquired in a focused state with respect to the eye to be examined, corresponding to each of a plurality of focal point distances of the intraocular lens.

According to such an aspect, the plurality of Hartmann images, each of which is acquired in a state of being focused on the eye to be examined corresponding to each of the plurality of focal point distances of the IOL, is acquired, and the dioptric power is calculated using each of the Hartmann images. Thereby, even when the eye E to be examined wearing the IOL has the function of accommodation, the reliability of the calculation results of the dioptric power of the eye E to be examined wearing the IOL can be improved.

In the ophthalmic information processing apparatus according to the fifth aspect of some embodiments, in any one of the first aspect to the fourth aspect, the intraocular lens information represents either a monofocal type or a multifocal type.

According to such an aspect, the reliability of the calculation result of the dioptric power of the eye to be examined wearing the monofocal type IOL or the multifocal type IOL can be improved.

In the ophthalmic information processing apparatus according to the sixth aspect of some embodiments, in the fifth aspect, when the intraocular lens is a monofocal type intraocular lens, the calculator is configured to calculate a single dioptric power based on the wavefront aberration information.

According to such an aspect, the reliability of the calculation result of the dioptric power of the eye to be examined wearing the IOL can be improved, depending on a plurality of types of IOL including the monofocal type IOL.

In the ophthalmic information processing apparatus according to the seventh aspect of some embodiments, in the fifth aspect, when the intraocular lens is a multifocal type intraocular lens, the calculator is configured to calculate a plurality of dioptric powers, each of the dioptric powers corresponding to each of the focal point distances of the intraocular lens, based on the wavefront aberration information.

According to such an aspect, the reliability of the calculation result of the dioptric power of the eye to be examined wearing the IOL can be improved, depending on a plurality of types of IOL including the multifocal type IOL.

In the ophthalmic information processing apparatus according to the eighth aspect of some embodiments, in the fifth aspect, the multifocal type includes a multifocal refractive type and a multifocal diffractive type.

According to such an aspect, the reliability of the calculation result of the dioptric power of the eye to be examined wearing the IOL can be improved, depending on a plurality of types of IOL including the multifocal refractive type IOL and the multifocal diffractive type.

In the ophthalmic information processing apparatus according to the ninth aspect of some embodiments, in the eighth aspect, when the intraocular lens is the multifocal refractive type intraocular lens, the calculator is configured to calculate a plurality of dioptric powers for each area corresponding to the focal point distance of the intraocular lens, based on the wavefront aberration information.

According to such an aspect, the dioptric power is calculated for each focal point distance (area) of the IOL. Thereby, the reliability of the calculation result of the dioptric power of the eye to be examined wearing the multifocal refractive type IOL can be improved.

In the ophthalmic information processing apparatus according to the tenth aspect of some embodiments, in the eighth aspect, the wavefront aberration information includes a Hartmann image. When the intraocular lens is the multifocal diffractive type intraocular lens, the calculator is configured to classify two or more separated point images for each focal point distance, the separated point image being generated by separating a point image that makes up the Hartmann image, and to calculate a plurality of dioptric powers for each focal point distance based on the classified two or more separated point images.

According to such an aspect, the two or more separated point images generated by separating the point image constituting the Hartmann image are classified for each focal point distance, and the plurality of dioptric powers is calculated for each focal point distance, based on the two or more separated point images that are classified. Thereby, the reliability of the calculation result of the dioptric power of the eye to be examined wearing the multifocal diffractive type IOL can be improved.

In the ophthalmic information processing apparatus according to the eleventh aspect of some embodiments, in the eighth aspect, the multifocal type further includes an extended depth of focus type. The wavefront aberration information includes a Hartmann image. When the intraocular lens is the extended depth of focus type intraocular lens, the calculator is configured to classify two focal points of an approximate ellipse for each focal point distance, the approximate ellipse being identified by performing elliptical approximation on each of a plurality of point images that make up the Hartmann image, and to calculate a plurality of dioptric powers for each focal point distance based on classified two or more separated point images.

According to such an aspect, the two focal point of the approximate ellipse, which is identified by performing elliptical approximation on each of the plurality of point images constituting the Hartmann image, are classified for each focal point distance, and the plurality of dioptric powers is calculated for each focal point distance based on the two or more separated point images. Thereby, the reliability of the calculation result of the dioptric power of the eye to be examined wearing the EDoF type IOL can be improved.

The ophthalmic information processing apparatus according to the twelfth aspect of some embodiments, in the fifth aspect, includes a display controller (controller 110, main controller 111) configured to display a dioptric power calculated by the calculator for each of a far point and a near point on a display means (display unit 170).

According to such an aspect, the highly reliable calculation result of the dioptric power of the eye to be examined wearing the IOL can be displayed on the display means.

The ophthalmic information processing apparatus according to the thirteenth aspect of some embodiments, in the fifth aspect, includes a display controller (controller 110, main controller 111) configured to display an image representing a view of the eye to be examined corresponding to a dioptric power calculated by the calculator on a display means (display unit 170), for each focal point distance of the intraocular lens or for each of one or more fixed distances.

According to such an aspect, the image representing a view of the eye to be examined corresponding to a dioptric power calculated in accordance with the type of IOL can be displayed on the display means for each focal point distance of the IOL or for each of one or more fixed distances.

The ophthalmic information processing apparatus according to the fourteenth aspect of some embodiments, in the fifth aspect, includes a display controller (controller 110, main controller 111) configured to display a simulation result of a visual acuity value of the eye to be examined on a display means (display unit 170), for each focal point distance of the intraocular lens or for each of one or more fixed distances.

According to such an aspect, the simulation result of the visual acuity value of the eye to be examined can be displayed for each focal point distance of the IOL or each of the one or more fixed distances. Thereby, the simulation result and the dioptric power calculated in accordance with the type of IOL can be easily compared.

The fifteenth aspect of some embodiments is an ophthalmic apparatus (100) including a measurement optical system (aberration measurement projection system 6 and aberration measurement light reception system 7) including a focusing lens (74) and configured to measure wavefront aberration of the eye to be examined; and the ophthalmic information processing apparatus of any one of the first aspect to the fourth aspect.

According to such an aspect, the ophthalmic apparatus capable of calculation the dioptric power of the eye to be examined wearing the IOL using a calculation processing method in accordance with the type of the IOL worn by the eye to be examined and of improving the reliability of the calculation result of the dioptric power of the eye to be examined wearing an IOL can be provided.

An ophthalmic information processing method according to the sixteenth aspect of some embodiments is the ophthalmic information processing method for calculating a dioptric power of an eye (E) to be examined based on wavefront aberration information obtained by performing wavefront aberration measurement on the eye to be examined wearing an intraocular lens. The ophthalmic information processing method includes an acquisition step of acquiring intraocular lens information representing at least optical characteristics of the intraocular lens, and a calculation step of changing calculation processing method of the dioptric power in accordance with the intraocular lens information to calculate the dioptric power of the eye to be examined.

According to such an aspect, the dioptric power of the eye to be examined wearing the IOL is calculated using a calculation processing method in accordance with the type of the IOL worn by the eye to be examined. Thereby, the reliability of the calculation result of the dioptric power of the eye to be examined wearing an IOL can be improved.

In the ophthalmic information processing method according to the seventeenth aspect of some embodiments, in the sixteenth aspect, the acquisition step is performed to acquire pupil diameter information representing a pupil diameter, and the calculation step is performed to calculate the dioptric power based on wavefront aberration information within a region demarcated based on the pupil diameter information.

According to such an aspect, the dioptric power is calculated based on the wavefront aberration information corresponding to the pupil diameter. Thereby, the reliability of the calculation result of the dioptric power of the eye to be examined wearing an IOL can be further improved.

In the ophthalmic information processing method according to the eighteenth aspect of some embodiments, in the sixteenth aspect, the wavefront aberration information includes a Hartmann image acquired in a focused state with respect to the eye to be examined, corresponding to an average focal point distance of the intraocular lens.

According to such an aspect, the reliability of the calculation result of the dioptric power of the eye to be examined wearing the IOL can be improved, with simple controls, depending on the type of IOL.

In the nineteenth aspect of some embodiments, in the sixteenth aspect, the wavefront aberration information includes a plurality of Hartmann images acquired in a focused state with respect to the eye to be examined, corresponding to each of a plurality of focal point distances of the intraocular lens.

According to such an aspect, the plurality of Hartmann images, each of which is acquired in a state of being focused on the eye to be examined corresponding to each of the plurality of focal point distances of the IOL, is acquired, and the dioptric power is calculated using each of the Hartmann images. Thereby, even when the eye E to be examined wearing the IOL has the function of accommodation, the reliability of the calculation results of the dioptric power of the eye E to be examined wearing the IOL can be improved.

In the ophthalmic information processing method according to the twentieth aspect of some embodiments, in any one of the sixteenth aspect to the nineteenth aspect, the intraocular lens information represents either a monofocal type or a multifocal type.

According to such an aspect, the reliability of the calculation result of the dioptric power of the eye to be examined wearing the monofocal type IOL or the multifocal type IOL can be improved.

In the ophthalmic information processing method according to the twenty-first aspect of some embodiments, in the twentieth aspect, when the intraocular lens is a monofocal type intraocular lens, the calculation step is performed to calculate a single dioptric power based on the wavefront aberration information.

According to such an aspect, the reliability of the calculation result of the dioptric power of the eye to be examined wearing the IOL can be improved, depending on a plurality of types of IOL including the monofocal type IOL.

In the ophthalmic information processing method according to the twenty-second aspect of some embodiments, when the intraocular lens is a multifocal type intraocular lens, the calculation step is performed to calculate a plurality of dioptric powers, each of the dioptric powers corresponding to each of the focal point distances of the intraocular lens, based on the wavefront aberration information.

According to such an aspect, the reliability of the calculation result of the dioptric power of the eye to be examined wearing the IOL can be improved, depending on a plurality of types of IOL including the multifocal type **IOL.**

A program according to the twenty-third aspect of some embodiments is the program of causing a computer to execute each step of the ophthalmic information processing method in any one of the sixteenth aspect to the nineteenth aspect.

According to such an aspect, the dioptric power of the eye to be examined wearing the IOL is calculated using a calculation processing method in accordance with the type of the IOL worn by the eye to be examined. Thereby, a program capable of improving the reliability of the calculation result of the dioptric power of the eye to be examined wearing an IOL can be provided.

### (Other modification examples)

The embodiment described above is merely an example for implementing the present invention. Those who intend to implement the present invention can apply any modification, omission, addition, or the like within the scope of the gist of the present invention.

It is possible to apply the invention according to the above embodiments to apparatuses having arbitrary functions adaptable in the field of ophthalmology. Examples of such functions include a tonometry function, a fundus photography function, an anterior segment photography function, an optical coherence tomography (OCT) function, an ultrasonic examination function, and the like. The intraocular pressure measurement function is realized by the tonometer, etc. The fundus imaging function is realized by the fundus camera, the scanning laser ophthalmoscope (SLO), or the like. The anterior segment imaging function is realized by the slit lamp, etc. The OCT function is realized by the OCT apparatus, etc. The ultrasonic inspection function is realized by the ultrasonic diagnosis apparatus, etc. Further, the present invention can also be applied to an apparatus (multifunctional apparatus) having two or more of such functions.

### [Explanation of symbols]

4 Optotype projection system
5 Observation system
6 Aberration measurement projection system
7 Aberration measurement light reception system
61, 61A, 61B Light source
75 Hartmann plate
76 Area sensor
100 Ophthalmic apparatus
110 Controller
111 Main controller
120 Arithmetic Processor
130 Dioptric power calculator
131 First dioptric power calculator
131A, 132A, 133B, 134A Point image identifying unit
131B, 132B, 133C Representative position identifying unit
131C, 132D, 133E, 134D Zernike polynomial approximate processor
132 Second dioptric power calculator
132C, 133D, 134C Point image group identifying unit
133 Third dioptric power calculator
133A Enhancement processor
134 Fourth dioptric power calculator
134B Elliptical approximate processor
140 Distribution information generator
150 Simulation processor
E Eye to be examined
Ef Fundus

## Claims

1. An ophthalmic information processing apparatus configured to calculate a dioptric power of an eye to be examined based on wavefront aberration information obtained by performing wavefront aberration measurement on the eye to be examined wearing an intraocular lens, the ophthalmic information processing apparatus comprising:
an acquisition unit configured to acquire intraocular lens information representing at least optical characteristics of the intraocular lens; and
a calculator configured to change calculation processing method of the dioptric power in accordance with the intraocular lens information to calculate the dioptric power of the eye to be examined.

2. The ophthalmic information processing apparatus of claim 1, wherein
the acquisition unit is configured to acquire pupil diameter information representing a pupil diameter, and
the calculator is configured to calculate the dioptric power based on wavefront aberration information within a region demarcated based on the pupil diameter information.

3. The ophthalmic information processing apparatus of claim 1, wherein
the wavefront aberration information includes a Hartmann image acquired in a focused state with respect to the eye to be examined, corresponding to an average focal point distance of the intraocular lens.

4. The ophthalmic information processing apparatus of claim 1, wherein
the wavefront aberration information includes a plurality of Hartmann images acquired in a focused state with respect to the eye to be examined, corresponding to each of a plurality of focal point distances of the intraocular lens.

5. The ophthalmic information processing apparatus of any one of claims 1 to 4, wherein
the intraocular lens information represents either a monofocal type or a multifocal type.

6. The ophthalmic information processing apparatus of claim 5, wherein
when the intraocular lens is a monofocal type intraocular lens, the calculator is configured to calculate a single dioptric power based on the wavefront aberration information.

7. The ophthalmic information processing apparatus of claim 5, wherein
when the intraocular lens is a multifocal type intraocular lens, the calculator is configured to calculate a plurality of dioptric powers, each of the dioptric powers corresponding to each of the focal point distances of the intraocular lens, based on the wavefront aberration information.

8. The ophthalmic information processing apparatus of claim 5, wherein
the multifocal type includes a multifocal refractive type and a multifocal diffractive type.

9. The ophthalmic information processing apparatus of claim 8, wherein
when the intraocular lens is the multifocal refractive type intraocular lens, the calculator is configured to calculate a plurality of dioptric powers for each area corresponding to the focal point distance of the intraocular lens, based on the wavefront aberration information.

10. The ophthalmic information processing apparatus of claim 8, wherein
the wavefront aberration information includes a Hartmann image, and
when the intraocular lens is the multifocal diffractive type intraocular lens, the calculator is configured to classify two or more separated point images for each focal point distance, the separated point image being generated by separating a point image that makes up the Hartmann image, and to calculate a plurality of dioptric powers for each focal point distance based on the classified two or more separated point images.

11. The ophthalmic information processing apparatus of claim 8, wherein
the multifocal type further includes an extended depth of focus type,
the wavefront aberration information includes a Hartmann image, and
when the intraocular lens is the extended depth of focus type intraocular lens, the calculator is configured to classify two focal points of an approximate ellipse for each focal point distance, the approximate ellipse being identified by performing elliptical approximation on each of a plurality of point images that make up the Hartmann image, and to calculate a plurality of dioptric powers for each focal point distance based on classified two or more separated point images.

12. The ophthalmic information processing apparatus of claim 5, further comprising
a display controller configured to display a dioptric power calculated by the calculator for each of a far point and a near point on a display means.

13. The ophthalmic information processing apparatus of claim 5, further comprising
a display controller configured to display an image representing a view of the eye to be examined corresponding to a dioptric power calculated by the calculator on a display means, for each focal point distance of the intraocular lens or for each of one or more fixed distances.

14. The ophthalmic information processing apparatus of claim 5, further comprising
a display controller configured to display a simulation result of a visual acuity value of the eye to be examined on a display means, for each focal point distance of the intraocular lens or for each of one or more fixed distances.

15. An ophthalmic apparatus, comprising:
a measurement optical system including a focusing lens and configured to measure wavefront aberration of the eye to be examined; and
the ophthalmic information processing apparatus of any one of claims 1 to 4.

16. An ophthalmic information processing method for calculating a dioptric power of an eye to be examined based on wavefront aberration information obtained by performing wavefront aberration measurement on the eye to be examined wearing an intraocular lens, the ophthalmic information processing method comprising:
an acquisition step of acquiring intraocular lens information representing at least optical characteristics of the intraocular lens; and
a calculation step of changing calculation processing method of the dioptric power in accordance with the intraocular lens information to calculate the dioptric power of the eye to be examined.

17. The ophthalmic information processing method of claim 16, wherein
the acquisition step is performed to acquire pupil diameter information representing a pupil diameter, and
the calculation step is performed to calculate the dioptric power based on wavefront aberration information within a region demarcated based on the pupil diameter information.

18. The ophthalmic information processing method of claim 16, wherein
the wavefront aberration information includes a Hartmann image acquired in a focused state with respect to the eye to be examined, corresponding to an average focal point distance of the intraocular lens.

19. The ophthalmic information processing method of claim 16, wherein
the wavefront aberration information includes a plurality of Hartmann images acquired in a focused state with respect to the eye to be examined, corresponding to each of a plurality of focal point distances of the intraocular lens.

20. The ophthalmic information processing method of any one of claims 16 to 19, wherein
the intraocular lens information represents either a monofocal type or a multifocal type.

21. The ophthalmic information processing method of claim 20, wherein
when the intraocular lens is a monofocal type intraocular lens, the calculation step is performed to calculate a single dioptric power based on the wavefront aberration information.

22. The ophthalmic information processing method of claim 20, wherein
when the intraocular lens is a multifocal type intraocular lens, the calculation step is performed to calculate a plurality of dioptric powers, each of the dioptric powers corresponding to each of the focal point distances of the intraocular lens, based on the wavefront aberration information.

23. A program of causing a computer to execute each step of the ophthalmic information processing method of any one of claims 16 to 19.
